# EUROPEAN PATENT SPECIFICATION

(11) **EP 1 686 989 B1**
(45) Date of publication and mention of the grant of the patent: **14.01.2009**
(21) Application number: 04798732.6
(22) Date of filing: 19.11.2004
(51) Int. Cl.: A61K 31/416, A61K 31/4162, A61K 31/55, A61K 31/695, A61P 25/00

(54) **USE OF 1,2-DIAZA-DIBENZO[E,H]AZULENES FOR THE MANUFACTURE OF PHARMACEUTICAL FORMULATIONS FOR THE TREATMENT AND PREVENTION OF CENTRAL NERVOUS SYSTEM DISEASES AND DISORDERS**
VERWENDUNG VON 1,2-DIAZADIBENZO[E,H]AZULENEN ZUR HERSTELLUNG VON PHARMAZEUTISCHEN FORMULIERUNGEN FÜR DIE BEHANDLUNG UND PRÄVENTION VON ERKRANKUNGEN UND STÖRUNGEN DES ZENTRALEN NERVENSYSTEMS
UTILISATION DE 1,2-DIAZA-DIBENZO[E,H] AZULENES POUR LA FABRICATION DE PREPARATIONS PHARMACEUTIQUES DESTINEES A TRAITER ET PREVENIR DES MALADIES ET DES TROUBLES DU SYSTEME NERVEUX CENTRAL

(30) Priority: 21.11.2003 HR 20030956
(43) Date of publication of application: 09.08.2006
(73) Proprietor: GlaxoSmithKline istrazivacki centar Zagreb d.o.o., 10000 Zagreb (HR)
(72) Inventor: MERCEP, Mladen, 10000 Zagreb (HR); MESIC, Milan, 10 000 Zagreb (HR); PESIC, Dijana, 22000 Sibenik (HR)
(74) Representative: von Füner, Nicolai
(86) International application number: PCT/HR2004/000053
(87) International publication number: WO 2005/049015

(56) References cited:
- EP-A- 0 063 525
- EP-A- 0 357 126
- WO-A-97/38991
- WO-A-03/099822
- OLIVERA, ROBERTO ET AL: "Revisiting the Ullmann-ether reaction: A concise and amenable synthesis of novel dibenzoxepino[4,5-d]pyrazoles by intramolecular etheration of 4,5-(o,o'-halohydroxy)arylpyrazoles" JOURNAL OF ORGANIC CHEMISTRY , 67(21), 7215-7225 CODEN: JOCEAH; ISSN: 0022-3263, 2002, XP008045270
- KAWASHIMA, KENYA ET AL: "Synthesis of dibenzo[b,f]cycloprop[d]azepine derivatives. III. Introduction of a cyclopropane ring by thermal decomposition of a pyrazoline" TAKEDA KENKYUSHOHO , 37(1-2), 6-11 CODEN: TAKHAA; ISSN: 0371-5167, 1978, XP009014897

## Description

The present invention relates to the use of compounds from the class of 1,2-diaza-dibenzo[e,h]azulenes as well as of their pharmacologically acceptable salts and solvates for the manufacture of a pharmaceutical formulation for the treatment and prevention of diseases and disorders of the central nervous system (CNS), wherein said diseases and disorders are selected from the group consisting of anxiety, depression and modest depression, bipolar disorders, sleeping disorders, sexual disorders, psychosis, borderline psychosis, schizophrenia, migraine, personality disorders and obsessive-compulsive disorders, social phobia or panic attacks, organic mental disorders in children, aggression, memory disorders and personality disorders in elderly people, addiction, obesity, bulimia, snoring, premenstrual troubles.

Irregularities in the steady state of biogenic amines (serotonin, norepinephrine, dopamine) and of other neurotransmitters and their receptors that are part of the central neurotransmitter system in CNS may be the cause of various mental diseases, damages and disorders (e.g. depression, schizophrenia, manic behavior and similar). Pathological changes in CNS caused by disorders of neurotransmitter concentration may occur due to an unbalanced (too big or too small) synthesis, irregularities in storing, releasing, metabolizing and/or reabsorption of biogenic amines and/or certain neurotransmitters.

The results of investigations directed to the understanding of the pathogenesis of mental disorders have shown that a disorder in the serotonin equilibrium plays an important role in various diseases. The monoamine-deficiency hypothesis was one of the first explanations, wherein the symptoms of depression were connected to a reduction in the neurotransmission of monoamines, especially serotonin (5-HT) and noradrenaline, which was also confirmed by neurochemical tests as well as by a successful treatment of the patients with substances increasing monoaminergic neurotransmission (Expert Opin. Investig. Drugs 2003, 12, 531-543). In addition to the serotonergic and noradrenergic systems, a very important role in CNS function disorders is also played by the dopaminergic system. The understanding of the exact role and of the interactions of these neurotransmitter systems is made rather difficult by the great number of receptor subtypes and their pharmacological complexity. Thus, it has been observed that e.g. dopaininergic neurotransmission is regulated by 5-HT_{2A} receptors (L. G. Spampinato, J. Neurochem. 2000, 74, 693-701) and hence 5-HT_{2A} receptors may also be the target receptors in treating diseases and disorders, in whose pathology an important role is played by a disorder of the function of the dopaminergic system (psychoses and various addictions).

Glutamate receptors play a vital role in the mediation of excitatory synaptic transmission as one of the major excitatory neurotransmitters in the central nervous system (CNS). It is widely accepted that σ1 receptor ligands can modulate neurotransmission mediated by central neurotransmitter systems, including glutamatergic/NMDA (F.P. Monnet, G. Debonnel, J.-L. Junien, C. de Montigny, Eur. J. Pharmacol.,1990, 179, 441-445). Many pharmacological and physiological actions have been attributed to the σ1 receptor. These include the regulation of IP3 receptors and calcium signaling at the endoplasmic reticulum, mobilization of cytoskeletal adaptor proteins, modulation of nerve growth factor-induced neurite sprouting, modulation of neurotransmitter release and neuronal firing, modulation of potassium channels as a regulatory subunit, alteration of psychostimulant-induced gene expression, and blockade of spreading depression. Behaviorally, the σ1 receptor is involved in learning and memory, psychostimulant-induced sensitization, cocaine-induced conditioned place preference, schizophrenia and pain perception. Thus, it is hypothesized that the σ1 receptor, at least in part, is an intracellular amplifier creating a supersensitized state for signal transduction in the biological system.

For treatment of pathological CNS disorders and particularly in the therapy of mental disorders a significant role as the most frequently applied medicines is given to substances that, according to their structure, are polycyclic compounds (benzodiazepines, tricyclic and tetracyclic antidepressants, monoamino oxidase (MAO) inhibitors, selective inhibitors of serotonin reabsorption).

A new area in pharmacotherapy was opened by introducing the novel tetracyclic antidepressant mianserin (Claghorn, J.; Lesem, M. D. Prog. Drug Res. 1996, 46, 243-262; Sperling, W.; Demling, J. Drugs Today 1997, 33, 95-102). Numerous tetracyclic derivatives showing pharmacological action in the treatment of the disorders of the neurochemical equilibrium in CNS are disclosed in the literature. WO 99/19317, WO 97/38991 and US 6,511,976 describe the manufacture of tetracyclic derivatives containing tetrahydrofuran ring and the use thereof as substances having antipsychotic, cardiovascular and gastrokinetic actions. US 4,145,434 discloses the manufacture of dibenzo(cyclohepta-, oxepino-, thiepino-)pyrrolidine and dibenzopyrrolidinoazepine derivatives as well as the use thereof as substances having a potential CNS action. The manufacture and a potential anxiolytic action of some tetracyclic isooxazolidine derivatives are disclosed as well (Drugs Fut. 2002, 27, Suppl. A: C41; Drugs Fut. 2002, 27, Suppl. A: P182, WO 96/14320, WO 96/14321). The introduction of a piperidine ring into a tetracyclic structure containing an oxepine ring resulted in the formation of the molecule Org-4428 showing an antidepressive action (Sperling, W.; Demling, J. Drugs Today 1997, 33, 95-102). The molecule Org-5222 contains a pyrrolidine ring fused to an oxepine nucleus and is described as a potential anxiolytic and antipsychotic (Sperling, W.; Demling, J. Drugs Today 1997, 33, 95-102). Some derivatives of 1,3-diaza-dibenzo[*e,h*]azulenes and salts thereof as a novel class of compounds with antiinflammatory action are known as well (US 3,711,489, US 4,198,421 and CA 967,573).

Known are also 2-substituted dibenzoazulenes of tetrahydropyrazole class with substituents such as acylalkyloxycarbonyl, phenyl or substituted phenyls (Gansser C. et al., Ann. Pharm. 1984, 41:465-471; or Olivera R. et al., Tetrahedron Letters, 2000, 41:4353-4356 4357-4360). Further there are known examples of dibenzoazepines of pyrazole class substituted in 2-position with alkyl (Kawashiha K Takeda Kenkysusho Ho 1978, 37:6-11, Fishou D et al., Tetrahedron 1984, 40:5121-5133), phenyl or substituted phenyl (FR 2,504,140, EP 0063525).

However, art known medicines used in the therapy of pathological CNS disorders and particularly in the therapy of mental disorders are associated with a wide range of adverse effects. There is thus a need for a safe and effective treatment of diseases and disorders of CNS.

In the earlier International publication WO 03/099822, compounds of 1,2-diaza-dibenzo[*e,h*]azulene class are disclosed, their pharmaceutically acceptable salts and solvates, process and intermediates for the preparation thereof as well as their antiinflammatory effects especially to the inhibition of tumor necrosis factor-α (TNF-α) production and the inhibition of interleukin-1 (IL-1) production along with their analgetic action.

EP 0 063 525 discloses tetracyclic compounds corresponding to the general formula 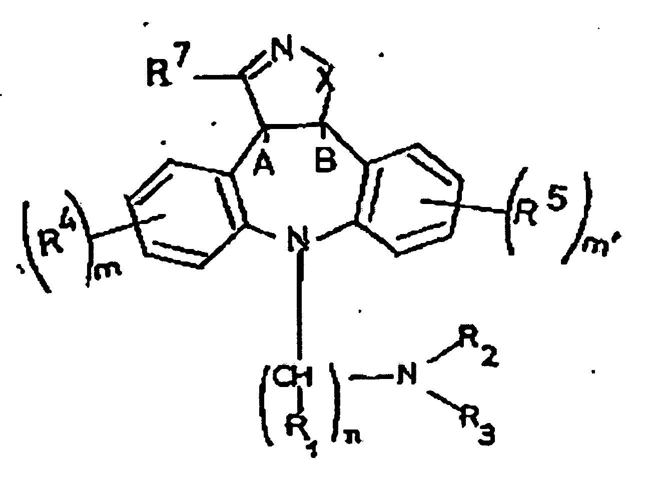 wherein R₁ represents hydrogen or lower alkyl, n is an integer from 1 to 5; R₂ represents hydrogen, lower alkyl, lower aralkyl or lower alkenyl; R₃ represents hydrogen, lower alkyl, lower aralkyl or lower alkenyl, or R₂ and R₃ together comprise an alkylene chain containing 2 to 6 carbon atoms optionally interrupted by 1 or 2 heteroatoms selected from the group consisting of oxygen, sulfur and a radical N-R₆ (R₆ being hydrogen, lower alkyl, lower hydroxyalkyl, lower alkoxyalkyl or lower acyloxyalkyl); R₄ and R₅ are independent from each other hydrogen, lower alkyl, halogen, trifluoromethyl, lower alkoxy, alkylenedioxy, hydroxy, thio, lower alkylthio, trichloromethoxy, trifluoromethoxy, trifluoromethylthio, amino, lower alkylamino, arylamino, lower alkylaminosulfonyl, morpholinosulfonyl, aminosulfonyl, cyano, nitro, carboxy, alkyloxycarbonyl, carbonamido, sulfinyl, sulfonyl, formyl or lower acyl; R₇ is lower alkyl, phenyl or phenyl optionally substituted by R₄; m and m' are independent from each other 1 to 3; A and B represent 2 hydrogen atoms or a double bond between two carbon atoms; X represents oxygen or a group N-R₈ (R₈ being phenyl substituted by one, two or three substituents or alkyl).

We have now surprisingly found that compounds from the class of 1,2-diaza-dibenzo[*e,h*]azulenes as described above are effective in the treatment of diseases and disorders of CNS. The present compounds are distinguished from the art-known tetracyclic compounds acting upon CNS (WO 99/19317, WO 97/38991; Sperling, W.; Demling, J. Drugs Today 1997, 33, 95-102, Olivera R. et al., Tetrahedron Letters, 2000, 41:4353-4356 4357-4360, Kawashiha K Takeda Kenkysusho Ho 1978, 37:6-11, EP 0063525) by valuable pharmacological and physicochemical properties.

According to our knowledge, the use of 1,2-diaza-dibenzo[*e,h*]azulenes and of their pharmaceutically acceptable salts and solvates disclosed in the earlier International publication WO 03/099822 for the manufacture of a pharmaceutical formulation for the treatment and prevention of diseases and disorders of the central nervous system, wherein said diseases and disorders are selected from the group consisting of anxiety, depression and modest depression, bipolar disorders, sleeping disorders, sexual disorders, psychosis, borderline psychosis, schizophrenia, migraine, personality disorders and obsessive-compulsive disorders, social phobia or panic attacks, organic mental disorders in children, aggression, memory disorders and personality disorders in elderly people, addiction, obesity, bulimia, snoring, premenstrual troubles has hitherto been neither disclosed nor suggested.

The subject-matter of the present invention is the use of compounds of the general formula **I** 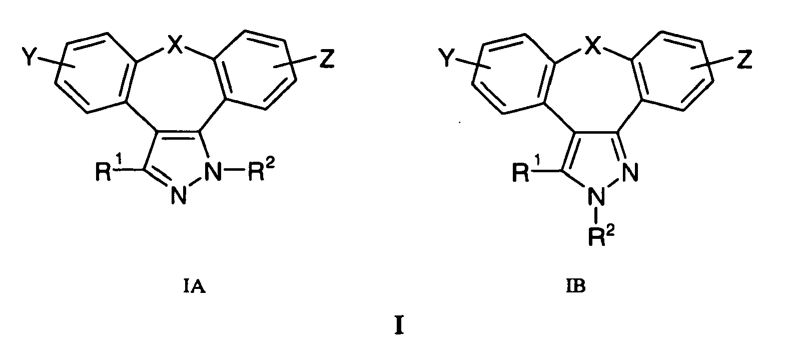 wherein
- X: means CH₂ or a heteroatom selected from a group consisting of O, S, S(=O), S(=O)₂ and NR^{a}, wherein R^{a} is hydrogen or a substituent selected from the group consisting of C₁-C₃-alkyl, C₁-C₃-alkanoyl, C₁-C₇-alkyloxycarbonyl, C₇- C₁₀-arylalkyloxycarbonyl, C₇-C₁₀-aroyl, C₇-C₁₀-arylalkyl, C₃-C₇ -alkylsilyl and C₅-C₁₀-alkylsilylalkyloxyalkyl;
- Y and Z: independently from each other mean one or more identical or different substituents linked to any available carbon atom selected from the group consisting of hydrogen, halogen, C₁-C₄-alkyl, C₂-C₄-alkenyl, C₂-C₄-alkinyl, halo-C₁-C₄-alkyl, hydroxy, C₁-C₄-alkoxy, trifluoromethoxy, C₁-C₄-alkanoyl, amino, amino-C₁-C₄-alkyl, *N*-(C₁*-*C₄-alkyl)amino, *N,N*-di(C₁-C₄-alkyl)amino, thiol, C₁-C₄-alkylthio, sulfonyl, C₁-C₄-alkylsulfonyl, sulfinyl, C₁-C₄- alkylsulfinyl, carboxy, C₁-C₄-alkoxycarbonyl, cyano, nitro;
- R¹: means CHO, CH₂OH or a substituent of the formula **II**: 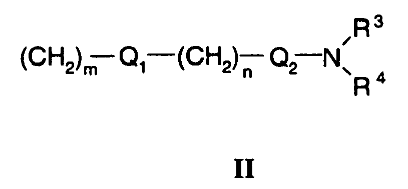 wherein
R³ and R⁴ simultaneously or independently from each other have the meaning of hydrogen, C₁-C₄-alkyl, aryl having the meaning of an aromatic ring as well as fused aromatic rings containing one ring with at least 6 carbon atoms or two rings with totally 10 carbon atoms and with alternating double bonds between carbon atoms; or together with N have the meaning of heterocycle or heteroaryl wherein heterocycle relates to five-membered or six-membered fully saturated or partly unsaturated heterocycle group containing at least one hetero atom selected from the group consisting of O, S and N and where said heterocycle can be optionally substituted with one or two substituents which are selected from halogen, C₁- C₄ alkyl, cyano, nitro, hydroxy, C₁-C₄ alkoxy, thiol, C₁-C₄ alkylthio, amino, *N*-(C₁-C₄-alkyl)amino, *N,N*-di(C₁-C₄- alkyl)amino, sulfonyl, C₁-C₄ alkylsulfonyl, sulfinyl, C₁-C₄ alkylsulfinyl; and wherein heteroaryl relates to aromatic and partially aromatic groups of a monocyclic or bicyclic ring with 4 to 12 carbon atoms and at least one of them being a heteroatom selected from the group consisting of O, S and N and where said heteroaryl can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁- C₄) alkylamino *N,N*-di(C₁-C₄-alkyl)-amino, sulfonyl, C₁-C₄ alkylsulfonyl, sulfinyl, C₁-C₄ alkylsulfinyl;
- m: represents an integer from 1 to 3;
- n: represents an integer from 0 to 3;
- Q₁ and Q₂: independently from each other have the meaning of oxygen, sulfur or a group: 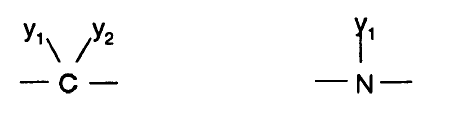 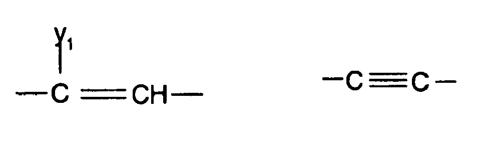 wherein substituents
y₁ and y₂ independently from each other have the meaning of hydrogen, halogen, C₁-C₄-alkyl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino, N,N-di(C₁-C₄-alkyl)-amino, sulfonyl, C₁-C₄ alkylsulfonyl, sulfinyl and C₁-C₄ alkylsulfinyl; aryl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino, N,N-di(C₁-C₄-alkyl)-amino, sulfonyl, C₁-C₄ alkylsulfonyl, sulfinyl and C₁-C₄ alkylsulfinyl wherein aryl has the meaning as defined above; hydroxy; C₁-C₄-alkoxy; C₁-C₄- alkanoyl; thiol; C₁-C₄-alkylthio; sulfonyl; C₁-C₄-alkylsulfonyl; sulfinyl; C₁-C₄-alkylsulfinyl; cyano; nitro; or together form a carbonyl or imino group; or
- **R**¹: has the meaning of hydrogen provided that simultaneously **R**² has the meaning of CH₂OCH₂CH₂Si(CH₃)₃, CH₂CH₂C₆H₅, CH₂CH₂OH or a substituent of the formula **II;**
- **R**²: means hydrogen, CH₂OCH₂CH₂Si(CH₃)₃, CH₂CH₂C₆H₅, CH₂CH₂OH or a substituent of the formula **II**, wherein formula **II** has the meaning as defined above;
and their pharmaceutically acceptable salts and solvates for the manufacture of pharmaceutical formulations for the treatment and prevention of diseases and disorders of the central nervous system, wherein said diseases and disorders are selected from the group consisting of anxiety, depression and modest depression, bipolar disorders, sleeping disorders, sexual disorders, psychosis, borderline psychosis, schizophrenia, migraine, personality disorders and obsessive-compulsive disorders, social phobia or panic attacks, organic mental disorders in children, aggression, memory disorders and personality disorders in elderly people, addiction, obesity, bulimia, snoring, premenstrual troubles.

The term "halo", "hal" or "halogen" relates to a halogen atom which may be fluorine, chlorine, bromine or iodine (most preferably chlorine or bromine).

The term "alkyl" relates to alkyl groups with the meaning of alkanes, which may be straight, branched or cyclic or a combination of straight and cyclic ones and branched and cyclic ones. The preferred straight or branched alkyls are e.g. methyl, ethyl, propyl, isopropyl, butyl, *sec*-butyl and *tert*-butyl. The preferred cyclic alkyls are e.g. cyclopentyl or cyclohexyl.

The term "haloalkyl" relates to alkyl groups which must be substituted with at least one halogen atom. The most frequent haloalkyls are e.g. chloromethyl, dichloromethyl, trifluoromethyl or 1,2-dichloropropyl.

The term "alkenyl" relates to alkenyl groups having the meaning of hydrocarbon radicals, which may be straight, branched or cyclic or are a combination of straight and cyclic ones or branched and cyclic ones, but having at least one carbon-carbon double bond. The most frequent alkenyls are ethenyl, propenyl, butenyl or cyclohexenyl.

The term "alkinyl" relates to alkinyl groups having the meaning of hydrocarbon radicals, which are straight or branched and contain at least one and at most two carbon-carbon triple bonds. The most frequent alkinyls are e.g. ethinyl, propinyl or butinyl.

The term "alkoxy" relates to straight or branched chains of alkoxy group. Examples of such groups are methoxy, propoxy, prop-2-oxy, butoxy, but-2-oxy or methylprop-2-oxy.

The term "aryl" relates to groups having the meaning of an aromatic ring, e.g. phenyl, as well as to fused aromatic rings. Aryl contains one ring with at least 6 carbon atoms or two rings with totally 10 carbon atoms and with alternating double (resonant) bonds between carbon atoms. The most frequently used aryls are e.g. phenyl or naphthyl. In general, aryl groups may be linked to the rest of the molecule by any available carbon atom via a direct bond or via a C₁-C₄ alkylene group such as methylene or ethylene.

The term "heteroaryl" relates to groups having the meaning of aromatic and partially aromatic groups of a monocyclic or bicyclic ring with 4 to 12 carbon atoms, at least one of them being a hetero atom such as O, S or N, and the available nitrogen atom or carbon atom is the binding site of the group to the rest of the molecule either via a direct bond or via a C₁-C₄ alkylene group defined earlier. Examples of this type are thiophenyl, pyrrolyl, imidazolyl, pyridinyl, oxazolyl, thiazolyl, pyrazolyl, tetrazolyl, pyrimidinyl, pyrazinyl, quinolinyl or triazinyl.

The term "heterocycle" relates to five-membered or six-membered, fully saturated or partly unsaturated heterocyclic groups containing at least one hetero atom such as O, S or N, and the available nitrogen atom or carbon atom is the binding site of the group to the rest of the molecule either via a direct bond or via a C₁-C₄ alkylene group defined earlier. The most frequent examples are morpholinyl, piperidinyl, piperazinyl, pyrrolidinyl, pyrazinyl or imidazolyl.

The term "alkanoyl" group relates to straight chains of acyl group such as formyl, acetyl or propanoyl.

The term "aroyl" group relates to aromatic acyl groups such as benzoyl.

The term "optionally substituted alkyl" relates to alkyl groups which may be optionally additionally substituted with one, two, three or more substituents. Such substituents may be halogen atom (preferably fluorine or chlorine), hydroxy, C₁-C₄ alkoxy (preferably methoxy or ethoxy), thiol, C₁-C₄ alkylthio (preferably methylthio or ethylthio), amino, *N*-(C₁₋C₄) alkylamino (preferably N-methylamino or *N*-ethylamino), *N,N*-di(C₁-C₄-alkyl)-amino (preferably dimethylamino or diethylamino), sulfonyl, C₁-C₄ alkylsulfonyl (preferably methylsulfonyl or ethylsulfonyl), sulfinyl, C₁-C₄ alkylsulfinyl (preferably methylsulfinyl).

The term "optionally substituted alkenyl" relates to alkenyl groups optionally additionally substituted with one, two or three halogen atoms. Such substituents may be e.g. 2-chloroethenyl, 1,2-dichloroethenyl or 2-bromo-propene-1-yl.

The term "optionally substituted aryl, heteroaryl or heterocycle" relates to aryl, heteroaryl or heterocyclic groups which may be optionally additionally substituted with one or two substituents. The substituents may be halogen (preferably chlorine or fluorine), C₁-C₄ alkyl (preferably methyl, ethyl or isopropyl), cyano, nitro, hydroxy, C₁-C₄ alkoxy (preferably methoxy or ethoxy), thiol, C₁-C₄ alkylthio (preferably methylthio or ethylthio), amino, *N*-(C₁-C₄) alkylamino (preferably *N*-methylamino or *N*-ethylamino), *N,N*-di(C₁-C₄-alkyl)-amino (preferably *N,N*-dimethylamino or *N,N-*diethylamino), sulfonyl, C₁-C₄ alkylsulfonyl (preferably methylsulfonyl or ethylsulfonyl), sulfinyl, C₁-C₄ alkylsulfinyl (preferably methylsulfinyl).

When X has the meaning of NR^{a}, R^{a} relates to hydrogen or a group selected from C₁-C₃-alkyl (preferably methyl or ethyl), C₁-C₃-alkanoyl (preferably formyl or acetyl), C₁-C₇-alkoxycarbonyl (preferably methoxycarbonyl or *tert*-butoxycarbonyl), C₇-C₁₀-arylalkyloxycarbonyl (preferably benzyloxycarbonyl), C₇-C₁₀-aroyl (preferably benzoyl), C₇-C₁₀-arylalkyl (preferably benzyl), C₃-C₇-alkylsilyl (preferably trimethylsilyl) or C₅-C₁₀-alkylsilylalkoxyalkyl (preferably trimethylsilylethoxymethyl).

When R³ and R⁴ together with N have the meaning of heteroaryl or heterocycle, this means that such heteroaryl or heterocycle has at least one carbon atom replaced by a nitrogen atom through which the groups are linked to the rest of the molecule. Examples of such groups are morpholine-4-yl, piperidine-1-yl, pyrrolidine-1-yl, imidazole-1-yl or piperazine-1-yl.

Depending upon the nature of particular substituents, the compounds of the formula **I** may have geometric isomers and one or more chiral centres so that there can exist enantiomers or diastereoisomers. The present invention also relates to the use of such isomers and mixtures thereof, including racemates.

The present invention also relates to all possible tautomeric forms of particular compounds of the formula **I**.

Whenever used hereinafter, the term "compounds of formula **I**" or "compounds of the present invention" is meant to also include the pharmaceutically acceptable addition salts and solvates.

In one embodiment of the present invention preferred compounds of formula **I** are those wherein X represents O, S, or NR^{a}, wherein R^{a} is hydrogen or a substituent selected from the group consisting of C₁-C₃-alkyl (preferably methyl, ethyl, propyl or isopropyl), C₁-C₃-alkanoyl (formyl or acetyl), C₇-C₁₀-aroyl (preferably benzoyl) and C₇-C₁₀-arylalkyl (preferably benzyl).

In another embodiment of the present invention preferred compounds of formula I are those wherein Y and Z independently from each other mean one or more identical or different substituents linked to any available carbon atom selected from the group consisting of hydrogen, fluorine, chlorine, bromine, C₁-C₄-alkyl (preferably methyl, ethyl, propyl or isopropyl), halo-C₁-C₄-alkyl (preferably trifluoromethyl), hydroxy, C₁-C₄-alkoxy (preferably methoxy), trifluoromethoxy, C₁-C₄-alkanoyl (preferably formyl or acetyl), amino, amino-C₁-C₄-alkyl (preferably aminomethyl), *N*-(C₁-C₄-alkyl)amino (preferably *N*-methyl or *N*-ethyl), *N,N*-di(C₁-C₄-alkyl)amino (preferably dimethylamino or diethylamino), thiol, C₁-C₄-alkylthio (preferably methylthio), cyano and nitro.

In yet another embodiment of the present invention preferred compounds of formula **I** are those wherein R¹ has the meaning of CHO, CH₂OH, or a substituent of the formula **II:** 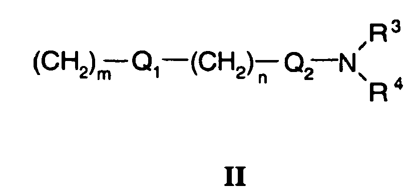 wherein
- R³ and R⁴: simultaneously or independently from each other have the meaning of hydrogen, C₁-C₄-alkyl (preferably methyl, ethyl, propyl or isopropyl), aryl wherein aryl has the meaning as defined above; or together with N have the meaning of heterocycle or heteroaryl selected from the group consisting of morpholine-4-yl, piperidine- 1-yl, pyrrolidine-1-yl, imidazole-1-yl and piperazine-1-yl;
- m: represents an integer from 1 to 3;
- n: represents an integer from 0 to 3;
- Q₁ and Q₂: independently from each other have the meaning of oxygen or CH₂ group; or

- R¹: has the meaning of hydrogen provided that simultaneously R² has the meaning of CH₂OCH₂CH₂Si(CH₃)₃, CH₂CH₂C₆H₅, CH₂CH₂OH or a substituent of the formula **II**.

In yet another embodiment of the present invention the specifically preferred compounds of formula **I** are:
*2-(8-oxa-1,2-diaza-dibenzo*[e,h]*azulene-1-yl)-ethanol;*
*2-(8-oxa-1,2-diaza-dibenzo*[e,h]*azulene-2-yl)-ethanol;*
*2-(8-thia-1,2-diaza-dibenzo*[e,h]*azulene-1-yl)-ethanol;*
*2-(8-thia-1,2-diaza-dibenzo*[e,h]*azulene-2-yl)-ethanol;*
*(2-phenethyl-2H-8-oxa-1,2-diaza-dibenzo*[e,h]*azulene-3-yl)-methanol;*
*(2-phenethyl-2H-8-thia-1,2-diaza-dibenzo*[e,h]*azulene-3-yl)-methanol;*
*[2-(2-trimethylsilyl-ethoxymethyl)*-2H*-8-oxa-1,2-diaza-dibenzo*[e,h]*azulene-3-yl]-methanol;*
*[2-(2-trimethylsilyl-ethoxymethyl)*-2H*-8-thia-1,2-diaza-dibenzo*[e,h]*azulene-3-yl]-methanol;*
*[11-chloro-2-(2-trimethylsilyl-ethoxymethyl)*-2H*-8-oxa-1,2-diaza-dibenzo*[e,h]*azulene-3-yl]-methanol;*
*dimethyl-{2-[2-(8-thia-1,2-diaza-dibenzo*[e,h]*azulen-1-yl)-ethoxy]-ethyl}-amine;*
*dimethyl-{3-[2-(8-thia-1,2-diaza-dibenzo*[e,h]*azulen-1-yl)-ethoxy]-propyl}-amine;*
*dimethyl-{2-[2-(8-thia-1,2-diaza-dibenzo*[e,h]*azulen-2-yl)-ethoxy]-ethyl}-amine;*
*dimethyl-{3-[2-(8-thia-1,2-diaza-dibenzo*[e,h]*azulen-2-yl)-ethoxy]-propyl}-amine;*
*dimethyl-[2-(2-phenethyl-*2H*-8-oxa-1,2-diaza-dibenzo*[e,h]*azulen-3-ylmethoxy)-ethyl]-amine;*
*dimethyl-[3-(2-phenethyl-*2H*-8-oxa-1,2-diaza-dibenzo*[e,h]*azulen-3-ylmethoxy)-propyl]-amine;*
*dimethyl-[2-(2-phenethyl-*2H*-8-thia-1,2-diaza-dibenzo*[e,h]*azulen-3-ylmethoxy)-ethyl]-amine;*
*dimethyl-[3-(2-phenethyl-*2H*-8-thia-1,2-diaza-dibenzo*[e,h]*azulen-3-ylmethoxy)-propyl]-amine;*
*dimethyl-{2-[2-(2-trimethylsilyl-ethoxymethyl)*-2H*-8-oxa-1,2-diaza-dibenzo*[e,h]*azulen-3-ylmethoxy]-ethyl}-amine;*
*dimethyl-[2-(1*H*-8-oxa-1,2-diaza-dibenzo*[e,h]*azulen-3-ylmethoxy)-ethyl]-amine; dimethyl-[2-(2*H*-8-oxa-1,2-diaza-dibenzo*[e,h]*azulen-3-ylmethoxy)-ethyl]-amine;*
*dimethyl-{3-[2-(2-trimethylsilyl-ethoxymethyl)-*2H*-8-oxa-1,2-diaza-dibenzo*[e,h]*azulen-3-ylmethoxy]-propyl}-amine;*
*dimethyl-[3-(1*H*-8-oxa-1,2-diaza-dibenzo*[e,h]*azulen-3-ylmethoxy)-propyl]-amine; dimethyl-[3-(2*H*-8-oxa-1,2-diaza-dibenzo*[e,h]*azulen-3-ylmethoxy)-propyl]-amine;*
*dimethyl-{2-[2-(2-trimethylsilyl-ethoxymethyl)-2*H*-8-thia-1,2-diaza-dibenzo*[e,h]*azulen-3-ylmethoxy]-ethyl}-amine;*
*dimethyl-[2-(1*H*-8-thia-1,2-diaza-dibenzo*[e,h]*azulen-3ylmethoxy)-ethyl]-amine; dimethyl-[2-(2*H*-8-thia-1,2-diaza-dibenzo*[e,h]*azulen-3-ylmethoxy)-ethyl]-amine;*
*dimethyl-{3-[2-(2-trimethylsilyl-ethoxymethyl)-2*H*-8-thia-1,2-diaza-dibenzo*[e,h]*azulen-3-ylmethoxy]-propyl}-amine;*
*dimethyl-[3-(1*H*-8-thia-1,2-diaza-dibenzo*[e,h]*azulen-3-ylmethoxy)-propyl]-amine; dimethyl-[3-(2*H*-8-thia-1,2-diaza-dibenzo*[e,h]*azulen-3-ylmethoxy)-propyl]-amine;*
*{2-[11-chloro-2-(2-trimethylsilyl-ethoxymethyl)-2H-8-oxa-1,2-diaza-dibenzo[e,h]azulen-3-ylmethoxy]-ethyl}-dimethyl-amine;*
*[2-(11-chloro-1*H*-8-oxa-1,2-diaza-dibenzo*[e,h]*azulen-3-ylmethoxy)-ethyl]-dimethylamine;*
*[2-(11-chloro-2*H*-8-oxa-1,2-diaza-dibenzo*[e,h]*azulen-3-ylmethoxy)-ethyl]-dimethylamine;*
*{3-[11-chloro-2-(2-trimethylsilyl-ethoxymethyl)-*2H*-8-oxa-1,2-diaza-dibenzo*[e,h]*azulen-3-ylmethoxy]-propyl}-dimethyl-amine,*
*[3-(11-chloro-1*H*-8-oxa-1,2-diaza-dibenzo*[e,h]*azulen-3-ylmethoxy)-propyl]-dimethylamine;* and
*[3-(11-chloro-*2H*-8-oxa-1,2-diaza-dibenzo*[e,h]*azulen-3-ylmethoxy)-propyl]-dimethylamine.*

Generally, the compounds of 1,2-diaza-dibenzo[e,h]azulene class, their pharmaceutically acceptable salts and solvates represented by the formula **I** can be prepared by the processes set forth in the earlier International publication WO 03/099822.

The compounds of the present invention are especially effective in treating those diseases and disorders as specified in the claims were the neurochemical equilibrium of biogenic amines such as serotonin, norepinephrine and dopamine was disturbed and which may be caused by unbalanced (too big or too small) synthesis, irregularities in storing, releasing, metabolizing and/or reabsorption of a certain neurotransmitter.

It has been found that the compounds of the present invention exhibit a significant binding affinity and have a high degree of selectivity to serotonin receptors, especially to 5-HT_{2A} and 5-HT_{2C}, as well as for σ1 receptor.

The compounds of formula **I**, or salt, or solvate thereof show binding affinity to 5-HT_{2A} and 5-HT_{2C} serotonin receptors in the concentration expressed as an IC₅₀ value less than 1 µM and having Kᵢ value less than 1 µM.

Especially, the compounds of formula **I**, or salt, or solvate thereof show binding affinity to 5-HT_{2A} serotonin receptor in the concentration expressed as an IC₅₀ value less than about 200 nM and having Kᵢ value less than about 100 nM.

Especially, the compounds of formula **I**, or salt, or solvate thereof show binding affinity to 5-HT_{2C} serotonin receptor in the concentration expressed as an IC₅₀ value less than about 200 nM and having Kᵢ value less than about 100 nM.

It has been found that the compounds of the present invention exhibit a significant binding affinity to σ1 receptor.

The compounds of formula **I**, or salt, or solvate thereof show binding affinity to σ1 receptor in the concentration expressed as an IC₅₀ value less than 1 µM and having Kᵢ value less than 1 µM.

Especially, the compounds of formula **I**, or salt, or solvate thereof show binding affinity to σ1 receptor in the concentration expressed as an IC₅₀ value less than about 200 nM and having Kᵢ value less than about 100 nM.

Since serotonin receptors are crucial in pathophysiology of a series of CNS disorders (directly or indirectly by participating in the activation of some other neurotransmitter e.g. dopamine and/or receptor), the compounds of the present invention may be used for the manufacture of pharmaceutical formulations for the treatment and prevention of diseases, damages and disorders, wherein biogenic amines and their receptors play an important role.

In view of the above explained favourable biological properties of the compounds of the present invention administration of the therapeutically effective amount of a compound of formula **I** provides an effective method of treatment of CNS diseases and disorders associated with fewer side effects due to their improved selectivity towards σ1 receptor and 5-HT_{2A} and 5-HT_{2C} serotonin receptors.

In general, the compounds of the present invention may be used for the manufacture of pharmaceutical formulations that are used as antidepressants, anxiolytics, antipsychotics or as drugs for treating migraine.

Likewise, these compounds may be used in the treatment and/or prevention of CNS damage caused by trauma, brain stroke, neurodegenerative diseases, cardiovascular disorders such as high blood pressure, thrombosis, infarct and similar diseases as well as by gastrointestinal disorders.

The effective dose of the active substance of the present invention and of a pharmaceutically acceptable salt or solvate thereof depends on the efficacy of the compound of the general formula **I**, on the nature and the severity of the disease and the disorder of CNS as well as on the body weight of the patient treated and may be from 0.001-10 mg/kg body weight. In any case a unit dose for an adult of an average weight of 70 kg is understood to be 0.07-1000 mg of the compound of the general formula **I** or of a pharmaceutically acceptable salt or solvate thereof. A unit dose may be administered once or several times daily, e.g. 2, 3 or 4 times daily, most frequently 1 to 3 times daily.

The present invention more specifically relates to an effective dose of the compounds, which bind to serotonin, sigma, adrenergic, dopamine or muscarinic receptors and/or act as inhibitors of reabsorption of one or more biogenic amines (serotonin, dopamine, norepinephrine).

The term "salts" can include acid addition salts or addition salts of free bases. Examples of acids which may be employed to form pharmaceutically acceptable acid addition salts include salts derived from nontoxic inorganic acids such as nitric, phosphoric, sulfuric, or hydrobromic, hydroiodic, hydrofluoric, phosphorous, as well as salts derived from nontoxic organic acids such as aliphatic mono- and dicarboxylic acids, phenyl-substituted alkanoic acids, hydroxyl alkanoic acids, alkanedioic acids, aromatic acids, aliphatic and aromatic sulfonic acids, and acetic, maleic, succinic, or citric acids. Examples of such salts include napadisylate, besylate, sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate. Also contemplated are salts of amino acids such as arginate and gluconate, galacturonate (see, for example, Berge S. M. et al. "Pharmaceutical Salts," J. of Pharma. Sci., 1977; 66:1).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium. Examples of suitable amines are N,N'-dibenzylethylenediamine, chloroprocaine, choline, diethanolamine, dicyclohexylamine, ethylenediamine, N-methylglucamine, and procaine.

The base addition salts of said acidic compounds are prepared by contacting the free acid form with a sufficient amount of the desired base to produce the salt in the conventional manner. The free acid form may be regenerated by contacting the salt form with an acid and isolating the free acid.

Preferred pharmaceutically acceptable salts according to the invention relate to salts of hydrobromic, hydrochloric, perchloric, sulfuric, maleic, fumaric, tartaric, citronic, benzoic, mandelic, methanesulfonic, benzenesulfonic, oxalic, p-toluenesulfonic, 2-naphthalenesulfonic and phosphoric acid.

Pharmaceutically acceptable solvates formed by the compounds represented by formula **I** or their salts relate to hydrates, ethanolates and similar (most frequently hydrates).

The phrase "pharmaceutically acceptable", as used in connection with compositions of the invention, refers to molecular entities and other ingredients of such compositions that are physiologically tolerable and do not typically produce untoward reactions when administered to a mammal (e.g., human). Preferably, as used herein, the term "pharmaceutically acceptable" means approved by a regulatory agency of the Federal or a state government or listed in the U.S. Pharmacopoeia or other generally recognized pharmacopeias for use in mammals, and more particularly in humans.

Further, the present invention relates to a pharmaceutical formulation containing an effective non-toxic dose of the compounds of the present invention as well as pharmaceutically acceptable carriers or solvents.

The term "carrier" applied to pharmaceutical compositions of the invention refers to a diluent, excipient, or vehicle with which an active compound is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil. However, since memantine is highly soluble, aqueous solutions are preferred. Suitable pharmaceutical carriers are described in "Remington's Pharmaceutical Sciences" by E.W. Martin, 18th Edition. Particularly preferred for the present invention are carriers suitable for immediate-release, i.e., release of most or all of the active ingredient over a short period of time, such as 60 minutes or less, and make rapid absorption of the drug possible.

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

The pharmaceutical formulations are obtained by blending a therapeutically active amount of a certain substance as the active ingredient with a pharmaceutically acceptable carrier, which may have different forms depending on the desired administration route. These pharmaceutical formulations especially relate to oral, sublingual, rectal, percutaneous or parenteral administration route.

Pharmaceutical formulations may be manufactured using conventional pharmaceutical auxiliaries and manufacture routes. Forms for oral administration may be syrups, capsules, tablets and similar forms where usual solid carriers are inert substances such as lactose, starch, glucose, methylcellulose, magnesium stearate, dicalcium phosphate, mannitol and usual liquid oral auxiliaries include ethanol, glycerol, water. All auxiliaries may be optionally blended with disintegrants, diluents, granulating agents, wetting agents, binders by using conventional methods. Parenteral forms may be manufactured by using water or some other sterile carrier. When for the manufacture of oral formulations some of the common liquid carriers e.g. water, glycol, oils, alcohols are used, the formulation may be in the form of syrup, emulsion, soft gelatine capsules or sterile injectable liquids e.g. ampoules, or of non-aqueous liquid suspensions. When for the manufacture of oral formulations a solid carrier such as starch, sugar, kaolin, wetting agents, binders, disintegrants is used, the formulation may be in the form of a powder, capsule, tablet, hard gelatine capsules or granules that may be administered in capsules, and the amount of the solid carrier may vary (most frequently from 1 mg to 1 g). Due to their easy use, tablets and capsules are the most convenient oral formulations wherein a solid carrier is used. For parenteral formulations the carrier is mostly sterile water, though other ingredients may be contained therein as well in order to improve solubility. For the manufacture of injectable solutions, sodium chloride solution, glucose solution or a mixture thereof is used. Injectable solutions may also contain a component for a delayed release of the active component. Convenient oils that may be used for this purpose are e.g. arachic oil, sesame oil, cottonseed oil, corn oil, soybean oil, synthetic glycerol esters of long-chain fatty acids or a mixture of some of said oils. Injectable suspensions may be manufactured in such a way that a suitable liquid carrier used is blended with a suspending agent. In formulations convenient for percutaneous administration, as a carrier there is understood a substance improving the penetration of the active substance and/or a suitable wetting agent, which may be combined with a suitable additive of any provenience, which additives do not cause harmful effects on skin. Said additives may facilitate the skin administration and/or may be used in the manufacture of the desired formulations, which may be applied in various ways e.g. transdermally, spot-on, or in the form of an ointment.

To improve the solubility and/or stability of the present compounds, in pharmacological formulations there may be used α-, β- or γ-cyclodextrins or derivatives thereof, especially hydroxyalkyl substituted cyclodextrins i.e. 2-hydroxypropyl-β-cyclodextrin. Cosolvents such as e.g. alcohols may also improve the solubility and/or stability of the present compounds in various pharmaceutical formulations.

"Treating" or "treatment" of a state, disorder or condition includes:
(1) preventing or delaying the appearance of clinical symptoms of the state, disorder or condition developing in a mammal that may be afflicted with or predisposed to the state, disorder or condition but does not yet experience or display clinical or subclinical symptoms of the state, disorder or condition,
(2) inhibiting the state, disorder or condition, i.e., arresting or reducing the development of the disease or at least one clinical or subclinical symptom thereof, or
(3) relieving the disease, i.e., causing regression of the state, disorder or condition or at least one of its clinical or subclinical symptoms.

The benefit to a subject to be treated is either statistically significant or at least perceptible to the patient or to the physician.

A "therapeutically effective amount" means the amount of a compound that, when administered to a mammal for treating a state, disorder or condition, is sufficient to effect such treatment. The "therapeutically effective amount" will vary depending on the compound, the disease and its severity and the age, weight, physical condition and responsiveness of the mammal to be treated.

Dosages and administration regimen can be adjusted depending on the age, sex, physical condition as well as the benefit acchieved by applying the compounds of the present invention and the side effects in the patient or the mammalian subject to be treated and the judgement of the physician, as is appreciated by those skilled in the art.

The term host or subject in need thereof as used herein refers to a mammal preferably a human.

The effect of the compounds of the present invention on the neurochemical steady state was determined by *in vitro* investigations such as a radionuclide-marked radioligand binding assay for 5-HT_{2A} (Bonhaus D.W. Br. J. Pharmacol. 1995, 115:622; Saucier C. J. Neurochem. 1997, 68:1998) and 5-HT_{2C} receptors (Wolf W.A. J. Neurochem. 1997, 69:1449), *in vitro* binding assay for σ1 receptor (Thomson W. and Donn R. Arthritis Res. 2002, 4: 302-306) and by *in vivo* investigations in a tail suspension test (Vogel H.G. and Vogel W.H. Drug Discovery and Evaluation Pharmacological Assays, Springer 1997, 304), in amphethamine-induced hyperlocomotion in mice (Millan M.J. et al, 1998 J Pharmacol. Exp. Ther. 287: 167-186), in a forced swim test in mice (Porsolt R.D. et al. Arch. Int. Pharmacodyn. 1977, 229:327-336), in meta-chlorophenyl piperazine (m-CPP) test on rats (Drug Dev. Res. 1989, 18:119-144), and in apomorphine, tryptamine, norepinephrine (ATN) test in rats (Arch. Int. Pharmacodyn. 1977, 227:238-253).

### In vitro method for determining affinity for binding to 5-HT_{2A} and 5-HT_{2C} receptors

A small concentration of a radioligand having a great affinity for binding to a receptor was incubated with a tissue sample enriched with a certain receptor (1-5 mg of tissue) in a buffered medium (0.2-5 mL). Recombinant human HT_{2A} and HT_{2C} receptors were expressed in CHO-K1 or COS-7 cells and were also used for competitive binding. During incubation the radioligand bound to the receptor. When a binding balance was achieved, the receptors to which the radioligand was bound were separated from those to which said ligand was not bound, and the radioactivity of the receptor/radioligand complex was measured. The interaction of the tested compounds with receptors was tested in competitive binding experiments. Various concentrations of tested compounds were added to the incubation mixture containing a prepared tissue enriched with corresponding receptors and the radioligand. The radioligand binding was inhibited by the test compounds proportionally to the affinity of a certain compound for the receptor and to the concentration of the compound.

The radioligand used for the determination of binding to 5-HT_{2A} receptor was [³H]-ketanserin and the tissue used was human cortex or recombinant 5-HT_{2A} receptor expressed in CHO-K1.

The radioligand used for the determination of binding to 5-HT_{2C} receptor was [³H]-mesulergine and the tissue used was choroid plexus or recombinant 5-HT_{2C} receptor expressed in CHO-K1 cells.

Compounds showing IC₅₀ and Kᵢ in concentrations lower than 1 µM, were considered to be active.

Compounds: [2-(11-chloro-1H-8-oxa-1,2-diaza-dibenzo[e,h]azulen-3-ylmethoxy)-ethyl]-dimethyl-amine, dimethyl-[3-(1H-8-thia-1,2-diaza-dibenzo[e,h]azulen-3-ylmethoxy)-propyl]-amine and dimethyl-[3-(2-phenethyl-2H-8-thia-1,2-diaza-dibenzo[e,h]azulen-3-ylmethoxy)-propyl]-amine showed binding affinity to 5-HT_{2A} and 5-HT_{2C} serotonin receptors expressed as IC₅₀ value less than 200 nM and Ki value less than 100 nM.

It is anticipated that similar results will be observed for other compounds of the invention.

### In vitro method for determining binding affinity to σ1 receptor

Jurkat cells were grown in medium, RPMI supplemented with 10% fetal bovine serum, 100U/ml penicillin and 100µg/ml streptomycin, collected and their suspension homogenized. After centrifugation, membrane fraction was separated, resuspended in phosphate buffer (pH=7.5) and stored in small aliquots in liquid nitrogen until use. Binding of different radiolabeled ligands to Jurkat cell membranes was measured as described previously (Ramamoorthy et al., 1995). To characterize the σ binding sites in the Jurkat cell line, [³H]haloperidol as first used as the ligand. Haloperidol is a high affinity ligand to both type 1 and type 2 σ-receptors. The binding assays were done using Jurkat cell membranes in the presence of [³H]haloperidol (10nM) alone to determine the total binding, and in the presence of [³H]haloperidol (10nM) and unlabeled haloperidol (10µM) to determine the nonspecific binding.

Membranes were incubated with ligands in phosphate buffer for 3 hours at room temperature. After filter had been washed, radioactivity associated with the filter was determined by liquid scintillation spectrometry.

Compounds showing IC₅₀ and Kᵢ in concentrations lower than 1 µM, were considered to be active.

It is anticipated that similar results will be observed for other compounds of the invention.

### Forced swim test in mice

Male CD1 mice of the weight of 20-25 g were used for the experiment. Groups of 10 animals were treated with the test compounds, imipramine (positive control) or the vehicle (negative control) by *per os* by gavage 30 min prior to testing to determine efficacy. On the day of the experiment the animals were placed into a glass cylinder (height 18.2 cm, diameter 13.3 cm) filled with water warmed to 22°C to the height of 10 cm. The immobility defined as the end of the struggling of the animal and the beginning of floating, wherein the movements were reduced to those indispensable for the animal to keep its head over the water surface, started to be recorded after two minutes and then it was monitored during 4 minutes.

The percentage of animals showing a passive behaviour was calculated and compared with a control group treated with a carrier.

The compounds that in a dose of 10 mg/kg reduced the immobility of animals for 30% and more over the control group were considered to be active.

It is anticipated that similar results will be observed for other compounds of the invention.

### Tail suspension test in mice

Male Balb/cJ mice of the weight of 20-25 g were used for the experiment. Groups of 9 animals were treated with the test compounds, imipramine (positive control) or the vehicle (negative control) by intraperitoneal injection, subcutaneous injection or per oral by gavage 30 min prior to testing to measure potential antidepressant activity. Mice were suspended from their tails at a height of about 90 cm and were observed for 5 minutes. The mice hanging fully motionless for 1 minute during the observation period were defined as depressive. In animals treated with a substance having an antidepressive action the period of immobility was shortened.

The percentage of animals showing a passive behaviour was calculated and compared with a control group treated with a vehicle. Significance of results was analysed using Fischer's exact test.

The compounds that in a dose of 10 mg/kg reduced the immobility of animals for 40% and more over a control group were considered to be active.

It is anticipated that similar results will be observed for other compounds of the invention.

### Amphetamine-induced hyperlocomotion in mice

Male Swiss OFA mice of a weight 30-35g were treated with either vehicle (saline) or test compounds 30 minutes prior to hyperlocomotion induction. Dexamphetamine sulphate was administered intraperitoneally at 2mg/kg. Thirty minutes later, animals were placed in a wooden box 80 x80 cm in a room with low light intensity (100 lux) for locomotor activity recording. Locomotor activity was determined during a 30 min period using a video image analyzer. Total duration of movement, occurence of movement and total distance travelled were measured. Haloperidol was tested at the dose of 0,25 mg/kg (prepared in 0,5% methylcellulose and served as reference substance.

Compounds were considered as active if in a dose of 10 mg/kg reduced amphethamine-induced hyperlocomotion in experimental animals for 30% and more when compared to vehicle treated control group.

It is anticipated that similar results will be observed for other compounds of the invention.

### Meta-chlorophenyl piperazine (m-CPP) test on rats

The tested substance was administered to rats per os 1 hour before the test and m-CPP in a dose of 1 mg/kg was administered intravenously 15 minutes before the test. At the beginning of the experiment the treated animals were subjected to an open field test on rats (Drug Dev. Res. 1989, 18, 119-144): the apparatus consisted of an open box having the dimensions 80 x 65 x 35 cm, which in one wall had an opening with a diameter of 10 cm, by which it was connected to a non-illuminated compartment having the dimensions 25 x 21 x 21 cm, and the opening was illuminated by a light source (IR source or Kleverlux^{®}; 12V/20W) from the distance of 66 cm; one hour after administering the tested substance, the animals were placed in the dark (non-illuminated) compartment so that their heads were turned away from the illuminated exit and the passing of the animals from the dark compartment to the bright one was measured for 10 minutes.

As an active dose of the substance there was defined a dose at which the effect induced by m-CPP was reduced for 40% and more.

It is anticipated that similar results will be observed for other compounds of the invention.

### Apomorphine, tryptamine, norepinephrine (ATN) test in rats

At the beginning of the experiment (t=0) the animals were injected intravenously by 1.25 mg/kg of apomorphine, then by 40 mg/kg of tryptamine (t=60 minutes) and by 1.25 mg/kg of norepinephrine (t=90 minutes).

There were watched a state of exceptional agitation and normal behaviour during 60 minutes (apomorphine test), then bilateral (two-sided) clonic convulsions of back paws (legs) and a general tremor of the body in tryptamine test (observation period 5 minutes) and lethality during 120 minutes after the injection in norepinephrine test.

The percentage of animals showing a passive behaviour was calculated and compared with a control group treated with a carrier.

The compounds which in a dose of 10 mg/kg reduced the period of duration of observed effects (mobility) for 40% over a control group were considered to be active in *in vivo* testings.

It is anticipated that similar results will be observed for other compounds of the invention.

Some of the present compounds tested in the above assays showed an action in at least two of said tests. These results represent an illustration of the biological action of the compounds.

## Claims

1. Use of the compounds of the general formula **I** 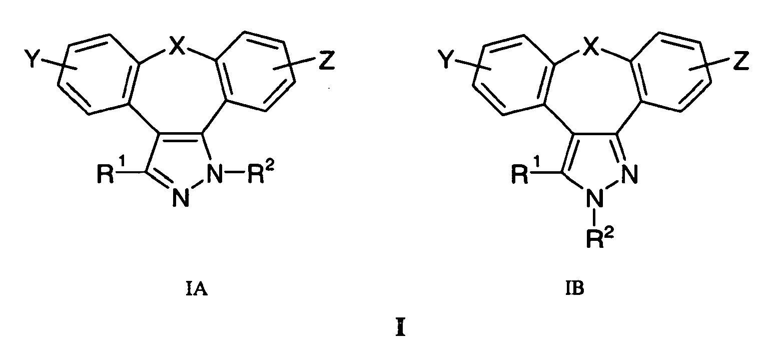 wherein
X means CH₂ or a heteroatom selected from a group consisting of O, S, S(=O), S(=O)₂ and NR^{a}, wherein R^{a} is hydrogen or a substituent selected from the group consisting of C₁-C₃-alkyl, C₁-C₃-alkanoyl, C₁-C₇-alkyloxycarbonyl, C₇- C₁₀-arylalkyloxycarbonyl, C₇-C₁₀-aroyl, C₇-C₁₀-arylalkyl, C₃-C₇ -alkylsilyl and C₅-C₁₀-alkylsilylalkyloxyalkyl;
Y and Z independently from each other mean one or more identical or different substituents linked to any available carbon atom selected from the group consisting of hydrogen, halogen, C₁-C₄-alkyl, C₂-C₄-alkenyl, C₂-C₄-alkinyl, halo-C₁-C₄-alkyl, hydroxy, C₁-C₄-alkoxy, trifluoromethoxy, C₁-C₄-alkanoyl, amino, amino-C₁-C₄-alkyl, *N*-(C₁-C₄-alkyl)amino, *N,N*-di(C₁-C₄-alkyl)amino, thiol, C₁-C₄-alkylthio, sulfonyl, C₁-C₄-alkylsulfonyl, sulfinyl, C₁-C₄- alkylsulfinyl, carboxy, C₁-C₄-alkoxycarbonyl, cyano and nitro;
R¹ means CHO, CH₂OH or a substituent of the formula **II**: 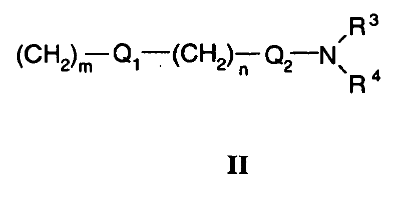
wherein
R³ and R⁴ simultaneously or independently from each other have the meaning of hydrogen, C₁-C₄-alkyl, aryl having the meaning of an aromatic ring as well as fused aromatic rings containing one ring with at least 6 carbon atoms or two rings with totally 10 carbon atoms and with alternating double bonds between carbon atoms; or together with N have the meaning of heterocycle or heteroaryl wherein heterocycle relates to five-membered or six-membered fully saturated or partly unsaturated heterocycle group containing at least one hetero atom selected from the group consisting of O, S and N and where said heterocycle can be optionally substituted with one or two substituents which are selected from halogen, C₁- C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)- amino, sulfonyl, C₁-C₄ alkylsulfonyl, sulfinyl, C₁-C₄ alkylsulfinyl; and wherein heteroaryl relates to aromatic and partially aromatic groups of a monocyclic or bicyclic ring with 4 to 12 carbon atoms and at least one of them being a heteroatom selected from the group consisting of O, S and N and where said heteroaryl can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁-C₄) alkylamino , N,N- di(C₁-C₄-alkyl)-amino, sulfonyl, C₁-C₄ alkylsulfonyl, sulfinyl, C₁- C₄ alkylsulfinyl;
m represents an integer from 1 to 3
n represents an integer from 0 to 3;
Q₁ and Q₂ independently from each other have the meaning of oxygen, sulfur or a group: 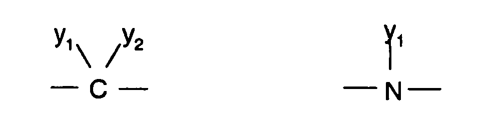 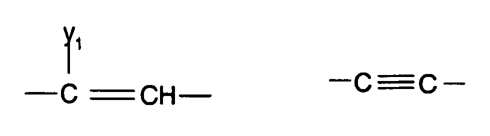 wherein substituents
y₁ and y₂ independently from each other have the meaning of hydrogen, halogen, C₁-C₄-alkyl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino, N,N-di(C₁-C₄-alkyl)-amino, sulfonyl, C₁-C₄ alkylsulfonyl, sulfinyl and C₁-C₄ alkylsulfinyl; aryl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino, N,N-di(C₁-C₄-alkyl)-amino, sulfonyl, C₁-C₄ alkylsulfonyl, sulfinyl and C₁-C₄ alkylsulfinyl wherein aryl has the meaning as defined above; hydroxy; C₁-C₄-alkoxy; C₁-C₄- alkanoyl; thiol; C₁-C₄-alkylthio; sulfonyl; C₁-C₄-alkylsulfonyl; sulfinyl; C₁-C₄-alkylsulfinyl; cyano; nitro, or together form a carbonyl or imino group; or
R¹ has the meaning of hydrogen provided that simultaneously R² has the meaning of CH₂OCH₂CH₂Si(CH₃)₃, CH₂CH₂C₆H₅, CH₂CH₂OH or a substituent of the formula **II**;
R² means hydrogen, CH₂OCH₂CH₂Si(CH₃)₃, CH₂CH₂C₆H₅, CH₂CH₂OH or a substituent of the formula **II**, wherein formula **II** has the meaning as defined above;
and their pharmaceutically acceptable salts and solvates for the manufacture of pharmaceutical formulations for the treatment and prevention of diseases and disorders of the central nervous system wherein said diseases and disorders are selected from the group consisting of anxiety, depression and modest depression, bipolar disorders, sleeping disorders, sexual disorders, psychosis, borderline psychosis, schizophrenia, migraine, personality disorders and obsessive-compulsive disorders, social phobia or panic attacks, organic mental disorders in children, aggression, memory disorders and personality disorders in elderly people, addiction, obesity, bulimia, snoring, premenstrual troubles.

2. Use according to claim 1, wherein the diseases and disorders of the central nervous system are selected from the group consisting of anxiety, depression, sleeping disorders, borderline psychosis and schizophrenia.

3. Use according to claim 1 wherein X represents O, S, or NR^{a} wherein R^{a} is hydrogen or a substituent selected from the group consisting of C₁-C₃-alkyl, C₁-C₃-alkanoyl, C₇-C₁₀-aroyl and C₇-C₁₀-arylalkyl.

4. Use according to claims 1 or 3 wherein Y and Z independently from each other mean one or more identical or different substituents linked to any available carbon atom selected from the group consisting of hydrogen, fluorine, chlorine, bromine, C₁-C₄-alkyl, halo-C₁-C₄-alkyl, hydroxy, C₁-C₄-alkoxy, trifluoromethoxy, C₁-C₄-alkanoyl, amino, amino-C₁-C₄-alkyl, C₁-C₄-alkylamino, *N*-(C₁-C₄-alkyl)amino, *N,N*-di(C₁-C₄-alkyl)amino, thiol, C₁-C₄-alkylthio, cyano and nitro.

5. Use according to claims 1, 3 or 4 wherein R¹ has the meaning of CHO, CH₂OH, or a substituent of the formula **II**: 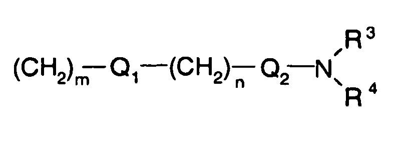 wherein
R³ and R⁴ simultaneously or independently from each other have the meaning of hydrogen, C₁-C₄-alkyl, aryl wherein aryl has the meaning as defined above; or together with N have the meaning of heterocycle or heteroaryl selected from the group consisting of morpholine-4- yl, piperidine-1-yl, pyrrolidine-1-yl, imidazole-1-yl and piperazine- 1-yl;
m represents an integer from 1 to 3;
n represents an integer from 0 to 3;
Q₁ and Q₂ independently from each other have the meaning of oxygen or a CH₂ group; or
R¹ has the meaning of hydrogen provided that simultaneously R² has the meaning of CH₂OCH₂CH₂Si(CH₃)₃, CH₂CH₂C₆H₅, CH₂CH₂OH or a substituent of the formula **II**.

6. Use according to claim 1, wherein the compounds of the general formula **I**, pharmaceutically acceptable salts and solvates thereof are selected from the group consisting of:
*2-(8-oxa-1,2-diaza-dibenzo*[e,h]*azulene-1-yl)-ethanol;*
*2-(8-oxa-1,2-diaza-dibenzo*[e,h]*azulene-2-yl)-ethanol;*
*2-(8-thia-1,2-diaza-dibenzo*[e,h]*azulene-1-yl)-ethanol;*
*2-(8-thia-1,2-diaza-dibenzo*[e,h]*azulene-2-yl)-ethanol;*
*(2-phenethyl-*2H*-8-oxa-1,2-diaza-dibenzo*[e,h]*azulene-3-yl)-methanol;*
*(2-phenethyl-*2H*-8-thia-1,2-diaza-dibenzo*[e,h]*azulene-3-yl)-methanol;*
*[2-(2-trimethylsilyl-ethoxymethyl)-*2H*-8-oxa-1,2-diaza-dibenzo*[e,h]*azulen-3-yl]-methanol;*
*[2-(2-trimethylsilyl-ethoxymethyl)-*2H*-8-thia-1,2-diaza-dibenzo*[e,h]*azulene-3-yl]-methanol;*
*[11-chloro-2-(2-trimethylsilyl-ethoxymethyl)-*2H*-8-oxa-1,2-diaza-dibenzo*[e,h]*azulene-3-yl]-methanol;*
*dimethyl-{2-[2-(8-thia-1,2-diaza-dibenzo*[e,h]*azulen-1-yl)-ethoxy]-ethyl}-amine;*
*dimethyl-{3-[2-(8-thia-1,2-diaza-dibenzo*[e,h]*azulen-1-yl)-ethoxy]-propyl}-amine;*
*dimethyl-{2-[2-(8-thia-1,2-diaza-dibenzo*[e,h]*azulen-2-yl)-ethoxy]-ethyl}-amine;*
*dimethyl-{3-[2-(8-thia-1,2-diaza-dibenzo*[e,h]*azulen-2-yl)-ethoxy]-propyl}-amine;*
*dimethyl-[2-(2-phenethyl-*2H*-8-oxa-1,2-diaza-dibenzo*[e,h]*azulen-3-ylmethoxy)-ethyl]-amine;*
*dimethyl-[3-(2-phenethyl-*2H*-8-oxa-1,2-diaza-dibenzo*[e,h]*azulen-3-ylmethoxy)-propyl]-amine;*
*dimethyl-[2-(2-phenethyl-*2H*-8-thia-1,2-diaza-dibenzo*[e,h]*azulen-3-ylmethoxy)-ethyl]-amine;*
*dimethyl-[3-(2-phenethyl-*2H*-8-thia-1,2-diaza-dibenzo*[e,h]*azulen-3-ylmethoxy)-propyl]-amine;*
*dimethyl-{2-[2-(2-trimethylsilyl-ethoxymethyl)-*2H*-8-oxa-1,2-diaza-dibenzo*[e,h]*azulen-3-ylmethoxy]-ethyl}-amine;*
*dimethyl-[2-(1*H*-8-oxa-1,2-diaza-dibenzo*[e,h]*azulen-3-ylmethoxy)-ethyl]-amine; dimethyl-[2-(2*H*-8-oxa-1,2-diaza-dibenzo*[e,h]*azulen-3-ylmethoxy)-ethyl]-amine;*
*dimethyl-{3-[2-(2-trimethylsilyl-ethoxymethyl)-2*H*-8-oxa-1,2-diaza-dibenzo*[e,h]*azulen-3-ylmethoxy]-propyl}-amine;*
*dimethyl-[3-(1*H*-8-oxa-1,2-diaza-dibenzo*[e,h]*azulen-3-ylmethoxy)-propyl]-amine; dimethyl-[3-(2*H*-8-oxa-1,2-diaza-dibenzo*[e,h]*azulen-3-ylmethoxy)-propyl]-amine;*
*dimethyl-{2-[2-(2-trimethylsilyl-ethoxymethyl)-*2H*-8-thia-1,2-diaza-dibenzo*[e,h]*azulen-3-ylmethoxy]-ethyl}-amine;*
*dimethyl-[2-(1*H*-8-thia-1,2-diaza-dibenzo*[e,h]*azulen-3-ylmethoxy)-ethyl]-amine; dimethyl-[2-(2*H*-8-thia-1,2-diaza-dibenzo*[e,h]*azulen-3-ylmethoxy)-ethyl]-amine;*
*dimethyl-{3-[2-(2-trimethylsilyl-ethoxymethyl)-*2H*-8-thia-1,2-diaza-dibenzo*[e,h]*azulen-3-ylmethoxy]-propyl}-amine;*
*dimethyl-[3-(1*H*-8-thia-1,2-diaza-dibenzo*[e,h]*azulen-3-ylmethoxy)-propyl]-amine; dimethyl-[3-(2*H*-8-thia-1,2-diaza-dibenzo*[e,h]*azulen-3-ylmethoxy)-propyl]-amine;*
*{2-[11-chloro-2-(2-trimethylsilyl-ethoxymethyl)-2H-8-oxa-1,2-diaza-dibenzo[e,h]azulen-3-ylmethoxyl-ethyl]-dimethyl-amine;*
*[2-(11-chloro-1*H*-8-oxa-1,2-diaza-dibenzo*[e,h]*azulen-3-ylmethoxy)-ethyl]-dimethylamine;*
*[2-(11-chloro-2*H*-8-oxa-1,2-diaza-dibenzo*[e,h]*azulen-3-ylmethoxy)-ethyl]-dimethylamine;*
*{3-[11-chloro-2-(2-trimethylsilyl-ethoxymethyl)-*2H*-8-oxa-1,2-diaza-dibenzo*[e,h]*azulen-3-ylmethoxy]-propyl}-dimethyl-amine,*
*[3-(11-chloro-1*H*-8-oxa-1,2-diaza-dibenzo*[e,h]*azulen-3-ylmethoxy)-propyl]-dimethylamine;* and
*[3-(11-chloro-*2H*-8-oxa-1,2-diaza-dibenzo*[e,h]*azulen-3-ylmethoxy)-propyl]-dimethylamine.*

## Patentansprüche

1. Verwendung der Verbindungen der allgemeinen Formel I 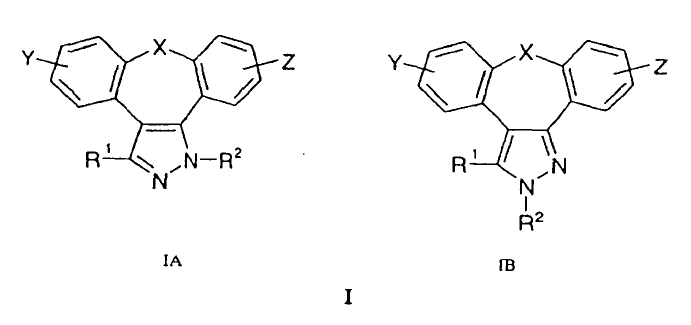 wobei
X CH₂ oder ein Heteroatom, ausgewählt aus O, S, S(=O), S(=O)₂ und NR^{a}, wobei R^{a} für Wasserstoff steht oder für einen Substituenten, ausgewählt aus C₁-C₃-Alkyl, C₁-C₃-Alkanoyl, C₁-C₇-Alkyloxycarbonyl, C₇-C₁₀-Arylalkyloxycarbonyl, C₇-C₁₀-Aroyl, C₇-C₁₀-Arylalkyl, C₃-C₇-Alkylsilyl und C₅-C₁₀-Alkylsilylalkyloxyalkyl, bedeutet;
Y und Z unabhängig voneinander einen oder mehrere identische oder verschiedene, an ein beliebiges verfügbares Kohlenstoffatom gebundene Substituenten, ausgewählt aus Wasserstoff, Halogen, C₁-C₄-Alkyl, C₂-C₄-Alkenyl, C₂-C₄-Alkinyl, Halo-C₁-C₄- alkyl, Hydroxy, C₁-C₄-Alkoxy, Trifluormethoxy, C₁-C₄-Alkanoyl, Amino, Amino- C₁-C₄-alkyl, N-(C₁-C₄-Alkyl)amino, N,N-Di(C₁-C₄-alkyl)amino, Thiol, C₁-C₄- Alkylthio, Sulfonyl, C₁-C₄-Alkylsulfonyl, Sulfinyl, C₁-C₄-Alkylsulfinyl, Carboxy, C₁-C₄-Alkoxycarbonyl, Cyano und Nitro, bedeuten;
R¹ CHO, CH₂OH oder einen Substituenten der Formel II: 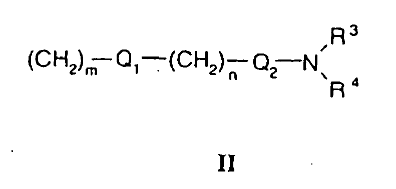
bedeutet, wobei
R³ und R⁴ gleichzeitig oder unabhängig voneinander die Bedeutung Wasserstoff, C₁-C₄-Alkyl, Aryl mit der Bedeutung eines aromatischen Rings sowie anellierter aromatischer Ringe, welche einen Ring mit mindestens 6 Kohlenstoffatomen oder zwei Ringe mit insgesamt 10 Kohlenstoffatomen und mit alternierenden Doppelbindungen zwischen den Kohlenstoffatomen enthält, haben; oder zusammen mit N die Bedeutung Heterocyclus oder Heteroaryl haben, wobei Heterocyclus sich auf einen fünfgliedrigen oder sechsgliedrigen vollständig gesättigten oder teilweise ungesättigten heterocyclischen Rest bezieht, welcher mindestens ein Heteroatom, ausgewählt aus O, S und N, enthält, und wobei der Heterocyclus gegebenenfalls mit einem oder zwei Substituenten, ausgewählt aus Halogen, C₁-C₄-Alkyl, Cyano, Nitro, Hydroxy, C₁-C₄-Alkoxy, Thiol, C₁-C₄-Alkylthio, Amino, N-(C₁-C₄)- Alkylamino, N,N-Di(C₁-C₄-alkyl)amino, Sulfonyl, C₁-C₄-Alkylsulfonyl, Sulfinyl, C₁-C₄-Alkylsulfinyl, substituiert sein kann; und wobei sich Heteroaryl auf aromatische und teilweise aromatische Reste eines monocyclischen oder bicyclischen Rings mit 4 bis 12 Kohlenstoffatomen bezieht und mindestens eines davon für ein Heteroatom, ausgewählt aus O, S und N steht und wobei Heteroaryl gegebenenfalls mit einem oder zwei Substituenten, ausgewählt aus Halogen, C₁-C₄-Alkyl, Cyano, Nitro, Hydroxy, C₁-C₄-Alkoxy, Thiol, C₁-C₄-Alkylthio, Amino, N-(C₁-C₄)-Alkylamino, N,N-Di(C₁-C₄-alkyl)amino, Sulfonyl, C₁-C₄-Alkylsulfonyl, Sulfinyl, C₁-C₄-Alkylsulfinyl, substituiert sein kann;
m eine ganze Zahl von 1 bis 3 darstellt;
n eine ganze Zahl von 0 bis 3 darstellt;
Q₁ und Q₂ unabhängig voneinander die Bedeutung Sauerstoff, Schwefel oder eines Rests: 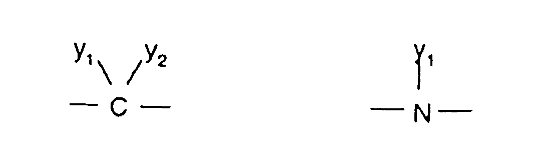 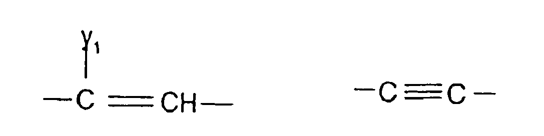
haben, wobei die Substituenten
y₁ und y₂ unabhängig voneinander die Bedeutung Wasserstoff, Halogen, C₁-C₄-Alkyl, gegebenenfalls substituiert mit einem, zwei, drei oder mehr Substituenten, ausgewählt aus Halogen, Hydroxy, C₁-C₄-Alkoxy, Thiol, C₁-C₄-Alkylthio, Amino, N-(C₁-C₄)-Alkylamino, N,N-Di(C₁-C₄-alkyl)amino, Sulfonyl, C₁-C₄-Alkylsulfonyl, Sulfinyl und C₁-C₄-Alkylsulfinyl; Aryl, gegebenenfalls substituiert mit einem, zwei, drei oder mehr Substituenten, ausgewählt aus Halogen, Hydroxy, C₁-C₄-Alkoxy, Thiol, C₁-C₄-Alkylthio, Amino, N-(C₁-C₄)-Alkylamino, N,N-Di(C₁-C₄-alkyl)amino, Sulfonyl, C₁-C₄-Alkylsulfonyl, Sulfinyl und C₁-C₄-Alkylsulfinyl, wobei Aryl die oben definierte Bedeutung hat; Hydroxy, C₁-C₄-Alkoxy; C₁-C₄-Alkanoyl; Thiol; C₁-C₄-Alkylthio; Sulfonyl, C₁-C₄-Alkylsulfonyl; Sulfinyl; C₁-C₄-Alkylsulfinyl; Cyano; Nitro haben, oder zusammen eine Carbonyl- oder Iminogruppe bilden; oder
R¹ die Bedeutung Wasserstoff hat, mit der Maßgabe, dass R² gleichzeitig die Bedeutung CH₂OCH₂CH₂Si(CH₃)₃, CH₂CH₂C₆H₅, CH₂CH₂OH oder eines Substituenten der Formel II hat;
R² die Bedeutung Wasserstoff, CH₂OCH₂CH₂Si(CH₃)₃, CH₂CH₂C₆H₅, CH₂CH₂OH oder eines Substituenten der Formel II hat, wobei die Formel II die oben definierte Bedeutung hat;
und deren pharmazeutisch verträgliche Salze und Solvate zur Herstellung pharmazeutischer Formulierungen zur Behandlung und Prävention von Krankheiten und Funktionsstörungen des zentralen Nervensystems, wobei die Krankheiten und Funktionsstörungen ausgewählt sind aus Angst, Depression und mäßiger Depression, bipolar affektiver Störungen, Schlafstörungen, Sexualstörungen, Psychose, Borderline-Psychose, Schizophrenie, Migräne, Persönlichkeitsstörungen und Zwangsstörungen, sozialer Phobie oder Panikattacken, organisch bedingten psychischen Störungen bei Kindern, Aggression, Gedächtnisstörungen und Persönlichkeitsstörungen bei älteren Menschen, Sucht, Fettleibigkeit, Bulimie, Schnarchen und prämenstrueller Beschwerden.

2. Verwendung gemäß Anspruch 1, wobei die Krankheiten und Funktionsstörungen des zentralen Nervensystems ausgewählt sind aus Angst, Depression, Schlafstörungen, Borderline-Psychose, und Schizophrenie.

3. Verwendung gemäß Anspruch 1, wobei X O, S oder NR^{a} darstellt, wobei R^{a} für Wasserstoff oder einen Substituenten, ausgewählt aus C₁-C₃-Alkyl, C₁-C₃-Alkanoyl, C₇-C₁₀-Aroyl und C₇-C₁₀-Arylalkyl, steht.

4. Verwendung gemäß Anspruch 1 oder 3, wobei Y und Z unabhängig voneinander einen oder mehrere identische oder verschiedene, an ein beliebiges verfügbares Kohlenstoffatom gebundene Substituenten, ausgewählt aus Wasserstoff, Fluor, Chlor, Brom, C₁-C₄-Alkyl, Halo-C₁-C₄-alkyl, Hydroxy, C₁-C₄-Alkoxy, Trifluormethoxy, C₁-C₄-Alkanoyl, Amino, Amino-C₁-C₄-alkyl, C₁-C₄-Alkylamino, N-(C₁-C₄-Alkyl)amino, N,N-Di(C₁-C₄-alkyl)amino, Thiol, C₁-C₄-Alkylthio, Cyano und Nitro, bedeuten.

5. Verwendung gemäß Anspruch 1, 3 oder 4, wobei R¹ die Bedeutung CHO, CH₂OH oder eines Substituenten der Formel II: 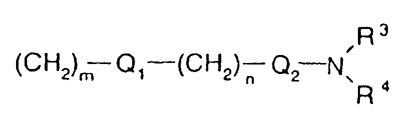 hat, wobei
R³ und R⁴ gleichzeitig oder unabhängig voneinander die Bedeutung Wasserstoff, C₁-C₄-Alkyl, Aryl, wobei Aryl die oben definierte Bedeutung hat, haben; oder zusammen mit N die Bedeutung Heterocyclus oder Heteroaryl, ausgewählt aus Morpholin-4-yl, Piperidin-1-yl, Pyrrolidin-1-yl, Imidazol-1-yl und Piperazin-1-yl, haben;
m eine ganze Zahl von 1 bis 3 darstellt;
n eine ganze Zahl von 0 bis 3 darstellt;
Q₁ und Q₂ unabhängig voneinander die Bedeutung Sauerstoff oder einer CH₂-Gruppe haben; oder
R¹ die Bedeutung Wasserstoff hat, mit der Maßgabe, dass gleichzeitig R² die Bedeutung CH₂OCH₂CH₂Si(CH₃)₃, CH₂CH₂C₆H₅, CH₂CH₂OH, oder eines Substituenten der Formel II hat.

6. Verwendung gemäß Anspruch 1, wobei die Verbindungen der allgemeinen Formel I, pharmazeutisch verträgliche Salze und Solvate davon ausgewählt sind aus:
2-(8-Oxa-1,2-diaza-dibenzo[e,h]azulen-1-yl)-ethanol;
2-(8-Oxa-1,2-diaza-dibenzo[e,h]azulen-2-yl)-ethanol;
2-(8-Thia-1,2-diaza-dibenzo[e,h]azulen-1-yl)-ethanol;
2-(8-Thia-1,2-diaza-dibenzo[e,h]azulen-2-yl)-ethanol;
(2-Phenethyl-2H-8-oxa-1,2-diaza-dibenzo[e,h]-azulen-3-yl)-methanol;
(2-Phenethyl-2H-8-thia-1,2-diaza-dibenzo[e,h]azulen-3-yl)-methanol;
[2-(2-Trimethylsilylethoxymethyl)-2H-8-oxa-1,2-diaza-dibenzo[e,h]azulen-3-yl]-methanol;
[2-(2-Trimethylsilylethoxymethyl)-2H-8-thia-1,2-diaza-dibenzo[e,h]azulen-3-yl]-methanol;
[11-Chlor-2-(2-trimethylsilylethoxymethyl)-2H-8-oxa-1,2-diaza-dibenzo[e,h]azulen-3-yl]-methanol;
Dimethyl-{2-[2-(8-thia-1,2-diaza-dibenzo[e,h]azulen-1-yl)-ethoxy]-ethyl} -amin;
Dimethyl-{3-[2-(8-thia-1,2-diaza-dibenzo[e,h]azulen-1-yl)-ethoxy]-propyl}-amin;
Dimethyl 2-[2-(8-thia-1,2-diaza-dibenzo[e,h]azulen-2-yl)-ethoxy]-ethyl -amin;
Dimethyl- {3-[2-(8-thia-1,2-diaza-dibenzo[e,h]azulen-2-yl)-ethoxy]-propyl}-amin;
Dimethyl-[2-(2-phenethyl-2H-8-oxa-1,2-diaza-dibenzo[e,h]azulen-3-ylmethoxy)-ethyl]-amin;
Dimethyl-[3-(2-phenethyl-2H-8-oxa-1,2-diaza-dibenzo[e,h]azulen-3-ylmethoxy)-propyl]-amin;
Dimethyl-[2-(2-phenethyl-2H-8-thia-1,2-diaza-dibenzo[e,h]azulen-3-ylmethoxy)-ethyl]-amin;
Dimethyl-[3-(2-phenethyl-2H-8-thia-1,2-diaza-dibenzo[e,h]azulen-3-ylmethoxy)-propyl]-amin;
Dimethyl- {2-[2-(2-trimethylsilylethoxymethyl)-2H-8-oxa- 1,2-diaza-dibenzo[e,h]azulen-3-ylmethoxy]-ethyl}-amin;
Dimethyl-[2-(1H-8-oxa-1,2-diaza-dibenzo[e,h]azulen-3-ylmethoxy)-ethyl]-amin;
Dimethyl-[2-(2H-8-oxa-1,2-diaza-dibenzo[e,h]azulen-3-ylmethoxy)-ethyl]-amin;
Dimethyl-{3-[2-(2-trimethylsilylethoxymethyl)-2H-8-oxa-1,2-diaza-dibenzo[e,h]azulen-3-ylmethoxy]-propyl}-amin;
Dimethyl-[3-(1H-8-oxa-1,2-diaza-dibenzo[e,h]azulen-3-ylmethoxy)-propyl]-amin;
Dimethyl-[3-(2H-8-oxa-1,2-diaza-dibenzo[e,h]azulen-3-ylmethoxy)-propyl]-amin;
Dimethyl- {2-[2-(2-trimethylsilylethoxymethyl)-2H-8-thia-1,2-diaza-dibenzo[e,h]azulen-3-ylmethoxy]-ethyl -amin;
Dimethyl-[2-(1H-8-thia-1,2-diaza-dibenzo[e,h]azulen-3-ylmethoxy)-ethyl]-amin;
Dimethyl-[2-(2H-8-thia-1,2-diaza-dibenzo[e,h]azulen-3-ylmethoxy)-eihyl]-amin;
Dimethyl-{3-[2-(2-trimethylsilylethoxymethyl)-2H-8-thia-1,2-diaza-dibenzo[e,h]azulen-3-ylmethoxy]-propyl}-amin;
Dimethyl-[3-(1H-8-thia-1,2-diaza-dibenzo[e,h]azulen-3-ylmethoxy)-propyl]-amin;
Dimethyl-[3-(2H-8-thia-1,2-diaza-dibenzo[e,h]azulen-3-ylmethoxy)-propyl]-amin;
{2-[11-Chlor-2-(2-trimethylsilylethoxymethyl)-2H-8-oxa-1,2-diaza-dibenzo[e,h]azulen-3-ylmethoxy]-ethyl}-diethylamin;
[2-(11-Chlor-1H-8-oxa-1,2-diaza-dibenzo[e,h]azulen-3-ylmethoxy)-ethyl]-dimethylamin;
[2-(11-Chlor-2H-8-oxa-1,2-diaza-dibenzo[e,h]azulen-3-ylmethoxy)-ethyl]-dimethylamin;
{3-[11-Chlor-2-(2-trimethylsilylethoxymethyl)-2H-8-oxa-1,2-diaza-dibenzo[e,h]azulen-3-ylmethoxy]-propyl}-dimethylamin;
[3-(11-Chlor-1H-8-oxa-1,2-diaza-dibenzo[e,h]azulen-3-ylmethoxy)-propyl]-dimethylamin; und
[3-(11-Chlor-2H-8-oxa-1,2-diaza-dibenzo[e,h]azulen-3-ylmethoxy)-propyl]-dimethylamin.

## Revendications

1. Utilisation des composés de formule générale I 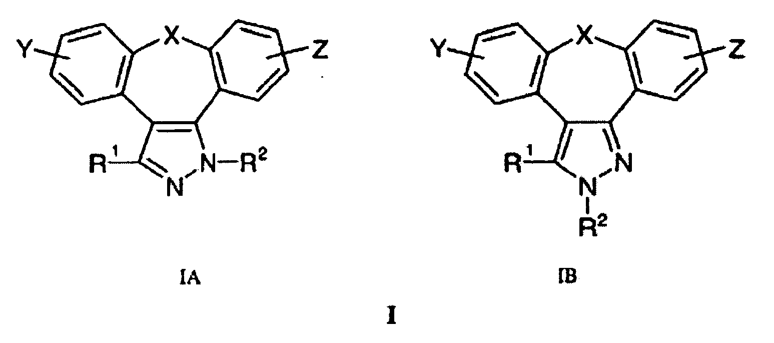 dans laquelle
X représente un groupe CH₂ ou un hétéroatome choisi dans un groupe consistant en O, S, S(=O), S(=O)₂ et NR^{a} dans lequel R^{a} représente un atome d'hydrogène ou un substituant choisi dans le groupe consistant en des substituants alkyle en C₁ à C₃, alcanoyle en C₁ à C₃, alkyloxycarbonyle en C₁ à C₇, arylalkyloxycarbonyle en C₇ à C₁₀, aroyle en C₇ à C₁₀, arylalkyle en C₇ à C₁₀, alkylsilyle en C₃ à C₇ et alkylsilylalkyloxyalkyle en C₅ à C₁₀ ;
Y et Z, indépendamment l'un de l'autre, représentent un ou plusieurs substituants identiques ou différents liés à n'importe quel atome de carbone disponible, choisis dans le groupe consistant en un atome d'hydrogène, un atome d'halogène, des groupes alkyle en C₁ à C₄, alcényle en C₂ à C₄, alcynyle en C₂ à C₄, halogénalkyle en C₁ à C₄, hydroxy, alkoxy en C₁ à C₄, trifluoro- méthoxy, alcanoyle en C₁ à C₄, amino, aminoalkyle en C₁ à C₄, *N-*(alkyle en C₁ à C₄) amino, *N,N*-di (alkyle en C₁ à C₄) amino, thiol, alkylthio en C₁ à C₄, sulfonyle, alkylsulfonyle en C₁ à C₄, sulfinyle, alkylsulfinyle en C₁ à C₄, carboxy, (alkoxy en C₁ à C₄) carbonyle, cyano et nitro ;
R¹ représente un groupe CHO, CH₂OH ou un substituant de formule II : 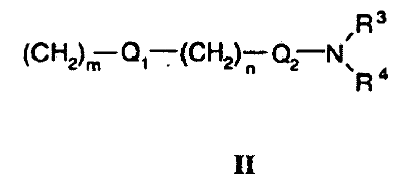
dans laquelle
R³ et R⁴, simultanément ou indépendamment l'un de l'autre, représentent un atome d'hydrogène, un groupe alkyle en C₁ à C₄, un groupe aryle représentant un noyau aromatique ainsi que des noyaux aromatiques condensés contenant un noyau ayant au moins 6 atomes de carbone ou deux noyaux ayant au total 10 atomes de carbone et avec des doubles liaisons alternées entre les atomes de carbone ; ou bien, conjointement avec l'atome de N, représentent un hétérocycle ou un groupe hétéroaryle, ledit hétérocycle représentant un groupe hétérocyclique penta- ou hexagonal totalement saturé ou partiellement insaturé contenant au moins un hétéroatome choisi dans le groupe consistant en O, S et N et ledit hétérocycle pouvant être facultativement substitué avec un ou plusieurs substituants qui sont choisis entre des substituants halogéno, alkyle en C₁ à C₄, cyano, nitro, hydroxy, alkoxy en C₁ à C₄, thiol, alkylthio en C₁ à C₄, amino, *N-*(alkyle en C₁ à C₄) amino, *N, N*-di (alkyle en C₁ à C₄)- amino, sulfonyle, alkylsulfonyle en C₁ à C₄, sulfinyle, et alkylsulfinyle en C₁ à C₄ ; et le groupe hétéroaryle représentant des groupes aromatiques et partiellement aromatiques constitués d'un noyau monocyclique ou bicyclique ayant 4 à 12 atomes de carbone, au moins un des atomes étant un hétéroatome choisi dans le groupe consistant en O, S et N et ledit groupe hétéroaryle pouvant être facultativement substitué avec un ou deux substituants qui sont choisis entre des substituants halogéno, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N,N*-di(alkyle en C₁ à C₄)- amino, sulfonyle, alkylsulfonyle en C₁ à C₄, sulfinyle et alkylsulfinyle en C₁ à C₄ ;
m représente un nombre entier de 1 à 3 ;
n représente un nombre entier de 0 à 3 ;
Q₁ et Q₂, indépendamment l'un de l'autre, représentent un atome d'oxygène, un atome de soufre, ou un groupe : 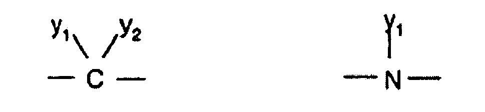 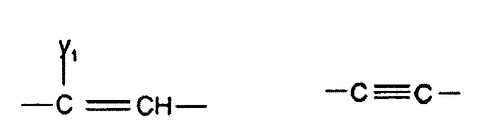 dans lequel les substituants
y₁ et y₂, indépendamment l'un de l'autre, représentent un atome d'hydrogène, un atome d'halogène, un groupe alkyle en C₁ à C₄ facultativement substitué avec un, deux, trois ou plus de trois substituants choisis dans le groupe consistant en un atome d'halogène, des substituants hydroxy, alkoxy en C₁ à C₄, thiol, alkylthio en C₁ à C₄, amino, N-(alkyle en C₁ à C₄) amino, N,N-di(alkyle en C₁ à C₄) amino, sulfonyle, alkylsulfonyle en C₁ à C₄, sulfinyle et alkylsulfinyle en C₁ à C₄ ; un groupe aryle facultativement substitué avec un, deux, trois ou plus de trois substituants choisis dans le groupe consistant en un atome d'halogène, des substituants hydroxy, alkoxy en C₁ à C₄, thiol, alkylthio en C₁ à C₄, amino, N-(alkyle en C₁ à C₄) amino, N,N-di-(alkyle en C₁ à C₄) amino, sulfonyle, alkylsulfonyle en C₁ à C₄, sulfinyle et alkylsulfinyle en C₁ à C₄, ledit groupe aryle répondant à la définition précité ; un groupe hydroxy ; alkoxy en C₁ à C₄ ; alcanoyle en C₁ à C₄ ; thio ; alkylthio en C₁ à C₄ ; sulfonyle ; alkylsulfonyle en C₁ à C₄ ; sulfinyle ; alkylsulfinyle en C₁ à C₄ ; cyano ou nitro ou bien forment conjointement un groupe carbonyle ou imino ; ou
R¹ représente un atome d'hydrogène, sous réserve que, simultanément, R² représente un groupe CH₂OCH₂CH₂Si(CH₃)₃. CH₂CH₂C₆H₅, CH₂CH₂OH ou un substituant de formule II ;
R² représente un atome d'hydrogène, un groupe CH₂OCH₂CH₂Si(CH₃)₃, CH₂CH₂C₆H₅, CH₂CH₂OH ou un substituant de formule II, la formule II répondant à la définition précitée ;
et de leurs sels et produits de solvatation pharmaceutiquement acceptables pour la production de formulations pharmaceutiques destinées au traitement et à la prévention de maladies et de troubles du système nerveux central, lesdites maladies et lesdits troubles étant choisis dans le groupe consistant en l'anxiété, la dépression et la dépression modérée, des troubles bipolaires, des troubles du sommeil, des troubles sexuels, la psychose, la psychose à la limite de la normale, la schizophrénie, la migraine, des troubles de la personnalité et des troubles obsessionnels compulsifs, la phobie sociale ou les attaques de panique, des troubles mentaux organiques chez l'enfant, l'agression, des troubles de la mémoire et des troubles de la personnalité chez les sujets âgés, la toxicomanie, l'obésité, la boulimie, les ronflements et les troubles prémenstruels.

2. Utilisation suivant la revendication 1, dans laquelle les maladies et troubles du système nerveux central sont choisis dans le groupe consistant en l'anxiété, la dépression, des troubles du sommeil, la psychose à la limite de la normale et la schizophrénie.

3. Utilisation suivant la revendication 1, dans laquelle X représente O, S ou un groupe NR^{a}, dans lequel R^{a} représente un atome d'hydrogène ou un substituant choisi dans le groupe consistant en des substituants alkyle en C₁ à C₃, alcanoyle en C₁ à C₃, aroyle en C₇ à C₁₀ et arylalkyle en C₇ à C₁₀ -

4. Utilisation suivant la revendication 1 ou 3, dans laquelle Y et Z, indépendamment l'un de l'autre, représentent un ou plusieurs substituants identiques ou différents liés à n'importe quel atome de carbone disponible, choisis dans le groupe consistant en des substituants hydrogène, fluor, chlore, brome, alkyle en C₁ à C₄, halogénalkyle en C₁ à C₄, hydroxy, alkoxy en C₁ à C₄, trifluorométhoxy, alcanoyle en C₁ à C₄, amino, aminoalkyle en C₁ à C₄, alkylamino en C₁ à C₄, *N-*(alkyle en C₁ à C₄)amino, *N, N*-di(alkyle en C₁ à C₄)amino, thiol, alkylthio en C₁ à C₄, cyano et nitro.

5. Utilisation suivant la revendication 1, 3 ou 4, dans laquelle R¹ représente un groupe CHO, CH₂OH ou un substituant de formule II : 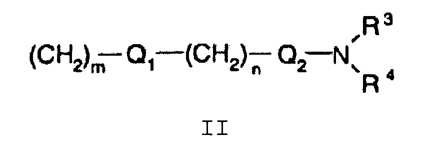 dans laquelle
R³ et R⁴, simultanément ou indépendamment l'un de l'autre, représentent un atome d'hydrogène, un groupe alkyle en C₁ à C₄, un groupe aryle, ledit groupe aryle répondant à la définition précitée ; ou bien, conjointement avec l'atome de N, représentent un hétérocycle ou un groupe hétéroaryle choisi dans le groupe consistant en les groupes morpholine-4-yle, pipéridine-1-yle, pyrrolidine- 1-yle, imidazole-1-yle et pipérazine-1-yle ;
m représente un nombre entier de 1 à 3 ;
n représente un nombre entier de 0 à 3 ;
Q₁ et Q₂, indépendamment l'un de l'autre, représentent un atome d'oxygène ou un groupe CH₂ ; ou bien
R¹ représente un atome d'hydrogène, sous réserve que, simultanément, R² représente un groupe CH₂OCH₂CH₂Si(CH₃)₃, CH₂CH₂C₆H₅, CH₂CH₂OH ou un substituant de formule II.

6. Utilisation suivant la revendication 1, dans laquelle les composés de formule générale I et leurs sels et leurs produits de solvatation pharmaceutiquement acceptables, sont choisis dans le groupe consistant en :
le *2-(8-oxa-1,2-diaza-dibenzo-*[e,h]*azulène-1-yl)-éthanol ;*
le *2-(8-oxa-2,2-diaza-dibenzo*[e,h]*azulène-2-yl)-éthanol ;*
le *2-(8-thia-1,2-diaza-dibenzo*[e,h]*azulène-1-yl)-éthanol ;*
le *2-(8-thia-1,2-diaza-dibenzo*[e,h]*azulène-2-yl)-éthanol ;*
le *(2-phénéthyl-2H-8-oxa-1,2-diaza-dibenzo*[e,h]*azulène-3-yl)-méthanol ;*
le *(2-phénéthyl-2H-8-thia-1,2-diaza-dibenzo*[e,h]*azulène-3-yl)-méthanol ;*
le *[2-(2-triméthylsilyl-éthoxyméthyl)*-2H*-8-oxa-1,2-diaza-dibenzo*[e,h]*azulène-3-yl]-méthanol ;*
le *[2-(2-triméthylsilyl-éthoxyméthyl)*-2H-*8-thia-1,2-diaza-dibenzo*[e,h]*azulène-3-yl]-méthanol ;*
le *[11-chloro-2-(2-triméthylsilyl-éthoxyméthyl)*-2H*-8-oxa-1,2-diaza-dibenzo*[e,h]*azulène-3-yl]-méthanol ;*
la *diméthyl-{2-[2-(8-thia-1,2-diaza-dibenzo*[e,h]*azulène-1-yl)-éthoxy]-éthyl}-amine ;*
la *diméthyl-{3-[2-(8-thia-1,2-diaza-dibenzo*[e,h]*azulène-1-yl)-éthoxy]-propyl}-amine ;*
la *diméthyl-{2-[2-(8-thia-1,2-diaza-dibenzo*[e,h]*azulène-2-yl)-éthoxy]-éthyl}-amine ;*
la *diméthyl-{3-[2-(8-thia-1,2-diaza-dibenzo*[e,h]*azulène-2-yl)-éthoxy]-propyl}-amine ;*
la *diméthyl-[2-(2-phénéthyl-2H-8-oxa-1,2-diaza-dibenzo*[e,h]-*azulène-3-ylméthoxy)-éthyl]-amine ;*
la *diméthyl-[3-(2-phénéthyl-2H-8-oxa-1,2-diaza-dibenzo*[e,h]-*azulène-3-ylméthoxy)-propyl]-amine ;*
la *diméthyl-[2-(2-phénéthyl-2H-8-thia-1,2-diaza-dibenzo*[e,h]-*azulène-3-ylméthoxy)-éthyl]-amine ;*
la *diméthyl-[3-(2-phénéthyl-2H-8-thia-1,2-diaza-dibenzo*[e,h]-*azulène-3-ylméthoxy)-propyl]-amine ;*
la diméthyl-(2-[2-(2-triméthylsilyl-éthoxyméthyl)-2H-8-oxa-*1,2-diaza-dibenzo*[e,h]*azulène-3-ylméthoxy]-éthyl)-amine ;*
la *diméthyl-[2-(1***H***-8-oxa-1,2-diaza-dibenzo*[e,h]*azulène-3-ylméthoxy)-éthyl]-amine ;*
la *diméthyl-[2-(2***H***-8-oxa-1,2-diaza-dibenzo*[e,h]*azulène-3-ylméthoxy)-éthyl]-amine ;*
la *diméthyl*-{3-[2-(2-triméthylsilyl-éthoxyméthyl)-2H-*8-oxa-1,2-diaza-dibenzo*[e,h]*azulène-3-ylméthoxy]-propyl}-amine ;*
la *diméthyl-[3-(1***H***-8-oxa-1,2-diaza-dibenzo*[e,h]*azulène-3-ylméthoxy)-propyl]-amine ;*
la *diméthyl-[3-(2***H***-8-oxa-1,2-diaza-dibenzo*[e,h]*azulène-3-ylméthoxy)-propyl]-amine ;*
la *diméthyl-{2-[2-(2-triméthylsilyl-éthoxyméthyl)-*2**H***-8-thia-1,2-diaza-dibenzo*[e,h]*azulène-3-ylméthoxy]-éthyl}-amine ;*
la *diméthyl-[2-(1***H***-8-thia-1,2-diaza-dibenzo*[e,h]*azulène-3-ylméthoxy)-éthyl]-amine ;*
la *diméthyl-[2-(2***H***-8-thia-1,2-diaza-dibenzo*[e,h]*azulène-3-ylméthoxy)-éthyl]-amine ;*
la *diméthyl-{3-[2-(2-triméthylsilyl-éthoxyméthyl)-2***H***-8-thia-1,2-diaza-dibenzo*[e,h]*azulène-3-ylméthoxy]-propyl}-amine ;*
la *diméthyl-[3-(1***H***-8-thia-1,2-diaza-dibenzo*[e,h]*azulène-3-ylméthoxy)-propyl]-amine ;*
la *diméthyl-[3-(2***H***-8-thia-1,2-diaza-dibenzo*[e,h]*azulène-3-ylméthoxy)-propyl]-amine ;*
la *{2-[11-chloro-2-(2-triméthylsilyl-éthoxyméthyl)-2H-8-oxa-1,2-diaza-dibenzo[e,h]azulène-3-ylméthoxy]-éthyl}-diméthylamine ;*
la *[2-(11-chloro-1H-8-oxa-1,2-diaza-dibenzo*[e,h]*azulène-3-ylméthoxy)-éthyl]-diméthylamine ;*
la *[2-(11-chloro-2H-8-oxa-1,2-diaza-dibenzo*[e,h]*azulène-3-ylméthoxy)-éthyl]-diméthylamine ;*
la *{3-[11-chloro-2-(2-triméthylsilyl-éthoxyméthyl)-2H-8-oxa-2,2-diaza-dibenzo*[e,h]*azulène-3-ylméthoxy]-propyl}-diméthylamine ;*
la *[3-(11-chloro-*1H*-8-oxa-1,2-diaza-dibenzo*[e,h]*azulène-3-ylméthoxy)-propyl]-diméthylamine ;* et
la *[3-(11-chloro-*2H*-8-oxa-1,2-diaza-dibenzo*[e,h]*azulène-3-ylméthoxy)-propyl]-diméthylamine.*
